# FASCICULE DE BREVET EUROPEEN

(11) **EP 3 129 782 B1**
(45) Date de publication et mention de la délivrance du brevet: **06.01.2021**
(21) Numéro de dépôt: 15713924.7
(22) Date de dépôt: 08.04.2015
(51) Int. Cl.: G01N 33/543, B01J 19/00, G01N 21/64

(54) **MARQUEUR DE CONTRÔLE POUR LA MISE EN OEUVRE DE PROCÉDÉS D'ANALYSE SUR SPOTS**
KONTROL-MARKER FÜR DIE UMSETZUNG VON SPOTS-BASIERENDEN ANALYSEVERFAHREN
CONTROL MARKER FOR IMPLEMENTING METHODS OF ANALYSIS ON SPOTS

(30) Priorité: 09.04.2014 FR 1453169
(43) Date de publication de la demande: 15.02.2017
(73) Titulaire: Bio-Rad Europe GmbH, 4051 Basel (CH)
(72) Inventeur: POUZET, Agnès Roseline Claude, 78210 Saint Cyr L'Ecole (FR); DOURY, Vincent, 92370 Chaville (FR); FOURNIER, Laurent Emmanuel, 78000 Versailles (FR); VÉDRINE, Christophe René Roger, 92400 Courbevoie (FR)
(74) Mandataire: Lavoix
(86) Numéro de dépôt international: PCT/EP2015/057639
(87) Numéro de publication internationale: WO 2015/155254

(56) Documents cités:
- US-A1- 2006 063 197
- US-A1- 2012 196 767

## Description

### Domaine de l'invention

La présente invention concerne l'utilisation d'un marqueur de contrôle pour la mise en œuvre de procédés d'analyse sur spots, en particulier dans le cadre d'analyses multiplexes. La présente invention a ainsi pour objet des supports solides contenant ledit marqueur de contrôle, leur procédé de préparation et leur utilisation dans des procédés d'analyse. La présente invention permet de vérifier la présence, la localisation et/ou l'intégrité des spots à l'issue du procédé d'analyse, et ainsi de sécuriser les résultats obtenus en garantissant que le résultat rendu résulte bien d'un spot présent, intègre et localisé.

### Etat de la technique

Un procédé d'analyse multiplexe permet de détecter, de manière simultanée, la présence éventuelle de plusieurs analytes au sein d'un même échantillon. Un procédé d'analyse multiplexe est typiquement mis en œuvre au moyen d'un support solide comprenant des spots, par exemple une microplaque comprenant des spots dans chaque puits, les spots étant chacun destinés à détecter un analyte ou à servir de contrôle.

Il est clair pour l'homme du métier qu'un risque lié à la technologie des spots est l'absence de dépôt, l'élimination ou la dégradation d'un ou plusieurs spots au cours du procédé de préparation du support solide, en particulier d'une microplaque, ou encore au cours de la mise en œuvre du procédé d'analyse au moyen dudit support solide. Le dispositif de dépôt des échantillons ou des réactifs peut, en effet, entrer accidentellement en contact avec un ou plusieurs spots, altérant ainsi leur surface, par exemple en formant une strie dans un ou plusieurs spots, ou en arrachant en partie ou totalement un ou plusieurs spots.

Par exemple, dans l'article de Bastarache et al. (Accuracy and Reproducibility of a multiplex Immunoassay platform : a validation study,, J.lmmunol Methods, Mar 31, 2011, 367 (1-2) 33-39*)* sont présentés des défauts qui ont été observés en fin de test sur le signal de luminescence, tels que des irrégularités de spot, la présence de comètes qui entraîne la contamination d'un spot par un spot voisin et l'absence de signal attendu à l'emplacement théorique du spot.

Or, lorsqu'un résultat négatif est rendu à l'issue d'un procédé d'analyse, ce résultat doit résulter de l'absence de l'analyte à détecter dans l'échantillon, et non d'une absence ou d'une dégradation du spot de détection de l'analyte correspondant. La sécurisation des procédés d'analyse est primordiale, notamment pour leur utilisation en diagnostic chez l'homme, par exemple pour vérifier l'absence de contamination virale ou bactérienne d'un échantillon sanguin en vue d'une transfusion.

La fabrication d'un support solide pour une analyse en spots consiste à déposer, à la surface du support solide, des solutions comprenant un ligand de capture de l'analyte à détecter, de manière à former des spots. Un contrôle de la qualité du support solide est ensuite effectué à l'issue de la fabrication du support solide, afin de ne garder que les supports solides présentant des spots intacts et bien formés.

Ainsi, le document US2006/0063197 décrit l'utilisation d'un fluorophore dans la solution de dépôt destinée à former les spots d'un microréseau. Le fluorophore est utilisé pour contrôler la qualité de chaque spot à l'issue du procédé de préparation du microréseau. D'ailleurs, seul un faible signal résiduel en fluorescence est détecté au niveau des spots avant l'ajout du substrat, au cours de la mise en œuvre d'un test ELISA.

Le document WO2012/142397 s'intéresse aux problématiques de flux dans les appareils microfluidiques contenant des microréseaux et décrit un assemblage de microréseau comprenant une chambre de réseau avec une entrée pour l'échantillon à une première extrémité, un microréseau et une sortie de l'échantillon à une deuxième extrémité reliée à une chambre de déchet, l'aire transversale à la première extrémité de la chambre de réseau étant plus large que celle à la deuxième extrémité. Ce document décrit également un procédé pour contrôler la qualité de fabrication d'un microréseau, en mesurant la fluorescence émise par un fluorophore de contrôle qualité interne au niveau des spots d'un réseau et en codant l'information relative à chaque spot du réseau sur un code barre, un dispositif mémoire ou en radio-identification, cette information ainsi codée étant associée au microréseau.

Par ailleurs, lors de la mise en œuvre d'un procédé d'analyse multiplexe, le signal correspondant à l'analyte à détecter peut être détecté au niveau d'une grille de lecture théorique. Cette grille de lecture théorique est généralement définie à la fin du procédé d'analyse multiplexe, à partir du signal détecté au niveau d'un spot servant de contrôle positif. Toutefois, compte tenu des échelles de lecture, tout décalage dans le positionnement des spots lors de la fabrication du support solide et/ou dans le positionnement du support solide dans le dispositif de détection du signal par rapport à la grille de lecture théorique entraîne un décalage dans le positionnement réel des spots par rapport à la grille de lecture théorique et a donc une influence sur la sensibilité de détection des analytes.

Il existe donc toujours un besoin de solutions pour sécuriser les résultats obtenus à l'issue d'un procédé d'analyse sur spots, en particulier en permettant de vérifier la présence, la localisation et/ou l'intégrité des spots à l'issue du procédé d'analyse et/ou en permettant d'optimiser la détection des analytes, par exemple en améliorant la sensibilité de détection des analytes.

### Description détaillée

La présente invention repose sur la mise en évidence de marqueurs de contrôle pouvant être déposés, pour la formation de spots sur un support solide, en même temps que des ligands de capture spécifiques des analytes à détecter dans un échantillon, ces marqueurs de contrôle étant capables de produire un signal à l'issue d'un procédé d'analyse mis en œuvre au moyen dudit support solide, sans interférer ou dans une mesure négligeable avec la détection des analytes. Les marqueurs de contrôle selon l'invention permettent ainsi de vérifier la présence, la localisation et/ou l'intégrité des spots à la fin d'un procédé d'analyse. Ces marqueurs de contrôle sont qualifiés ici de « marqueurs de contrôle résistants ».

Les expressions « procédé d'analyse » et « procédé de détection d'au moins un analyte » sont ici synonymes.

Les termes « produire », « produit » ou « production » s'appliquent à tout type de signal, et en particulier à tout type de rayonnement électromagnétique, qu'il s'agisse d'un signal radioactif, d'une émission lumineuse ou d'absorbance.

Lorsque le signal détecté est un signal en fluorescence ou en luminescence, le « signal produit » est en particulier un « signal émis », et par « produire un signal » et « production d'un signal» on entend alors « émettre un signal » et « émission d'un signal» respectivement.

Par « détection d'au moins un analyte » (ou « détecter au moins un analyte »), on entend, dans la présente demande, détection de la présence (ou détecter la présence) dudit ou desdits analyte(s) et/ou quantification dudit ou desdits analyte(s) (ou quantifier ledit ou lesdits analyte(s)).

Par « fin d'un procédé d'analyse », en entend, au sens de l'invention, après que les spots aient été mis en présence de l'échantillon à analyser, d'un ligand ou de ligands de détection d'un analyte, le cas échéant d'au moins un (premier) rapporteur d'un marqueur de détection couplé à un ligand de détection d'un analyte et, le cas échéant, d'au moins un (deuxième) rapporteur d'un marqueur de détection couplé audit premier rapporteur.

De manière surprenante, les inventeurs ont ainsi mis en évidence des marqueurs de contrôle résistants qui peuvent être fixés sur un support solide, qui n'interfèrent pas avec le procédé d'analyse lui-même et qui restent parfaitement détectables (en particulier car ils restent fixés au moins partiellement sur le support solide) après la mise en œuvre des différentes étapes du procédé d'analyse. Lorsque le signal produit par le marqueur de contrôle résistant à l'issue du procédé d'analyse permet de délimiter un spot répondant aux critères de qualité d'un spot, en particulier présence, localisation et/ou intégrité, le signal correspondant à un analyte peut alors être détecté au niveau de ce spot. En particulier, les marqueurs de contrôle résistants selon l'invention peuvent être utilisés dans un procédé d'analyse dans lequel la présence d'au moins un analyte est détectée par un signal émis en chimiluminescence, de préférence via la réaction d'une enzyme peroxydase avec un substrat luminol et/ou un analogue du luminol, par exemple l'isoluminol, et/ou un de leurs dérivés.

Les marqueurs de contrôle résistants selon l'invention présentent l'avantage de permettre le contrôle des spots d'un support solide à la fin d'un procédé d'analyse, et non uniquement à la fin du procédé de préparation dudit support solide.

Ainsi, la présente invention permet de garantir les résultats rendus à l'utilisateur. En particulier, la présente invention permet de garantir qu'un résultat négatif résulte bien de l'absence de l'analyte dans un échantillon et non par exemple d'une absence de spot ou d'un décalage dans la lecture du spot. En d'autres termes, la présente invention permet de supprimer des résultats faussement négatifs (appelés également « faux négatifs ») qui seraient liés à un défaut d'un spot (c'est à dire un spot non conforme aux critères de qualité) et/ou de lecture d'un spot en fin du procédé d'analyse et qui ne sont pas détectables dans les procédés d'analyse multiplexe classiquement utilisés, dans lesquels la grille de lecture est ajustée de manière théorique, par exemple sur le signal émis par un spot contrôle positif. La présente invention permet également de garantir qu'un résultat positif résulte bien de la présence de l'analyte dans un échantillon. Par exemple, il peut arriver de détecter un signal correspondant au marqueur d'un analyte à l'emplacement théorique d'un spot (tel que défini par une grille de lecture théorique, par exemple ajustée sur le signal émis par un spot contrôle), alors qu'il n'y a pas de spot à cet emplacement ; un résultat faussement positif (appelé également « faux positif ») serait alors rendu lors de la mise en œuvre d'un procédé d'analyse classique; en utilisant un marqueur de contrôle résistant selon l'invention dans les spots du support solide, aucun signal correspondant audit marqueur de contrôle résistant ne sera détecté au niveau de cet emplacement théorique d'un spot et le procédé d'analyse conduira à une absence de rendu de résultat, malgré la détection d'un signal correspondant au marqueur de l'analyte à cet emplacement.

Un autre avantage de la présente invention réside en ce que les marqueurs de contrôle résistants permettent de définir la grille de lecture à la fin du procédé d'analyse. Or, il peut y avoir des différences entre une grille de lecture théorique définie d'après les paramètres physiques du support solide et celle définie à l'issue d'un procédé d'analyse utilisant ledit support solide. Ces différences, peuvent par exemple résulter d'un décalage entre la grille de spots théorique attendue et la grille réellement obtenue à l'issue du procédé d'analyse (pour un ou plusieurs spots) ; ce décalage est ainsi corrigé par le dispositif décrit dans la présente demande. Il est ainsi plus fiable de définir la grille de lecture d'un support solide à l'issue du procédé d'analyse, de préférence avec le même dispositif de détection que celui utilisé pour détecter le signal produit par le marqueur de détection d'au moins un ligand de détection d'un analyte et de préférence de façon concomitante. Ainsi, les marqueurs de contrôle résistants selon l'invention permettent également de sécuriser les résultats d'un procédé d'analyse sur spots, en améliorant la sensibilité de la détection des analytes grâce à la définition d'une grille de lecture pour la détection des analytes à partir de la localisation des spots détectés à la fin du procédé d'analyse.

Enfin, la préparation d'un support solide dont les spots comprennent un marqueur de contrôle résistant selon l'invention est simple de mise en œuvre et n'ajoute pas d'étape supplémentaire, le marqueur de contrôle résistant pouvant être, par exemple, simplement mélangé avec le composé d'intérêt, tel qu'un ligand de capture, dans la solution à déposer pour former le spot.

La présente invention a particulièrement pour objet un support solide pour une détection sécurisée d'au moins un analyte dans un échantillon, dont au moins un spot destiné à la détection d'un analyte comprend au moins un marqueur de contrôle résistant et au moins un ligand de capture d'un analyte, un procédé de préparation d'un tel support solide et un procédé de détection sécurisée d'au moins un analyte dans au moins un échantillon au moyen dudit support solide.

Par « au moins un», on entend, dans la présente demande, un ou plusieurs, « plusieurs » signifiant en particulier deux, trois, quatre, cinq, six, sept, huit, neuf, dix, onze, douze, treize, quatorze, quinze ou plus de quinze. De façon similaire, par « au moins x », on entend, dans la présente demande, x ou plus de x, et en particulier x+1, x+2, x+3, x+4, *etc.,* « x » étant un nombre entier supérieur ou égal à 2, par exemple 2 ou 3.

Un procédé d'analyse particulièrement préféré selon l'invention est un procédé d'analyse multiplexe.

La présente invention a également pour objet un procédé de sélection d'un marqueur de contrôle résistant, ainsi que l'utilisation d'au moins un marqueur de contrôle résistant dans au moins un spot destiné à détecter un analyte pour sécuriser un procédé de détection d'au moins un analyte dans un échantillon.

La présente invention fournit également un dispositif approprié pour une détection améliorée d'au moins un analyte dans un échantillon.

### Echantillon

L'échantillon à analyser est, de préférence, un échantillon biologique.

L'échantillon biologique peut être un fluide biologique, tel qu'un échantillon de sang, dérivé de sang (tel que plasma ou sérum), urine, fluide céphalorachidien, salive ou leurs combinaisons, ou un échantillon de tissu, tel qu'un tissu obtenu par biopsie, une cellule, un ensemble de cellules, un extrait végétal, ou leurs combinaisons.

Un dérivé de sang désigne tout produit, en particulier fluide, obtenu à partir d'un échantillon de sang.

L'échantillon à analyser peut également être un milieu de culture et/ou un surnageant de culture.

Avant d'être analysé, l'échantillon peut subir une ou plusieurs étapes préalables de traitement, tels qu'une dilution, centrifugation, traitement thermique, lyse cellulaire, solubilisation, dénaturation (par exemple par un ou plusieurs agents chaotropiques, un ou plusieurs agents réducteurs et/ou par chauffage), extraction, réaction de PCR (*Polymerase Chain Reaction*), ajout d'un ligand de détection non marqué ou leurs combinaisons. L'ajout d'un ligand de détection non marqué est en particulier utile pour la mise en œuvre d'un test de neutralisation.

L'échantillon peut également être un mélange d'au moins deux échantillons qui peuvent être de même nature ou de nature différente.

A titre d'exemple de mélange d'échantillons de nature différente, on peut citer un mélange de sang et de sérum, un mélange de sang et de plasma, un mélange de sérum et de plasma, ou encore un mélange de sang, sérum et plasma.

Un échantillon préféré selon l'invention est un échantillon ou un mélange d'échantillons de sang et/ou dérivé(s) du sang (en particulier plasma et/ou sérum).

### Analyte

Un analyte à détecter dans l'échantillon peut être tout type de composé, naturel ou synthétique, que l'on souhaite détecter et / ou quantifier dans un échantillon.

Un analyte peut par exemple être une protéine, un peptide, une glycoprotéine, un glucide, une cellule, un organelle, un virus ou un acide nucléique.

La cellule peut être une cellule animale, une cellule végétale, une cellule de bactérie, un protozoaire, une cellule de métazoaire, une cellule de levure ou une cellule de champignon.

Un acide nucléique désigne un polymère de nucléotides reliés par des liaisons phosphodiester, tel qu'un acide désoxyribonucléique (ADN), un acide ribonucléique (ARN) ou un analogue de ceux-ci, tels que des phosphorothioates ou des thioesters, sous forme simple brin ou double brin.

L'analyte ou au moins un des analytes est ainsi par exemple choisi dans le groupe consistant en un antigène, un anticorps, un fragment d'anticorps, un haptène, une hormone, un récepteur d'hormone, une enzyme, ou un acide nucléique.

Le ou les analytes sont, de préférence, sélectionnés dans le groupe consistant en un antigène, un anticorps, un fragment d'anticorps, un haptène, une hormone, un récepteur d'hormone et une enzyme.

Par « antigène », on entend au sens de la présente demande une molécule naturelle, recombinante ou synthétique reconnue par des anticorps ou des cellules du système immunitaire et capable, lorsqu'elle est présentée dans des conditions appropriées au système immunitaire d'un hôte, d'induire une réponse immunitaire.

Un antigène peut être une molécule, en particulier un polypeptide, comprenant ou consistant en au moins un épitope qui peut être linéaire ou conformationnel. Le terme « épitope linéaire » désigne un polypeptide, en particulier un peptide, comprenant ou consistant généralement en 3 à 15 acides aminés, plus généralement 5 à 15 acides aminés, de préférence au moins 6, 8, 10 ou 12 acides aminés, capable de se lier à une molécule d'anticorps dirigé contre ledit antigène. Le terme « épitope conformationnel » désigne une structure tridimensionnelle reconnue par un anticorps et déterminée par la juxtaposition de plusieurs acides aminés dans l'espace, qui peuvent être non contigus dans la séquence peptidique de la protéine (ou du polypeptide) contre laquelle est dirigé cet anticorps, mais qui, du fait du repliement de la chaîne polypeptidique, se retrouvent proches les uns des autres dans l'espace, et peuvent ainsi former un motif susceptible d'être reconnu par un anticorps.

Un antigène au sens de l'invention est, par exemple, une protéine (en particulier une protéine native ou une protéine recombinante), un peptide (par exemple un peptide synthétique), une glycoprotéine, un glucide ou un lipide ; ledit peptide peut être associé ou non à une molécule porteuse.

Par « molécule porteuse » (appelée également molécule support), on entend notamment une molécule support protéique ou glucidique, en particulier une protéine support. Une molécule support peut être un polypeptide (en particulier une protéine ou un peptide) naturel ou non naturel (par exemple une protéine recombinante ou un peptide synthétique), un polymère fonctionnalisé (de type dextran, polysaccharide ou poly-lysine), un co-polymère mixte (en particulier un co-polymère d'acides aminés différents, par exemple un co-polymère lysine-tyrosine). Une molécule support peut être un anticorps (en particulier un anticorps monoclonal ou un anticorps polyclonal), par exemple une immunoglobuline (appelée également Ig).

Un exemple de molécule ou de protéine support est la BSA (*albumine sérique bovine*)*.*

Par « haptène », on désigne dans la présente demande une molécule de faible poids moléculaire capable d'être reconnue par le système immunitaire, mais qui est immunogène uniquement lorsqu'elle est couplée à une molécule porteuse.

Un analyte est, de préférence, un composé permettant de diagnostiquer une condition chez un sujet, pathologique ou non, ou de diagnostiquer les risques de développer une condition, pathologique ou non. Un exemple de condition non pathologique est une grossesse.

Le sujet peut être un homme, un animal non-humain ou un végétal. L'animal non-humain est de préférence un mammifère, tel qu'un chat, chien, singe, lapin, souris ou rat.

Le terme « homme » est utilisé de manière large et désigne notamment un homme ou une femme de tout âge, tel qu'un nourrisson, un enfant, un adolescent, un adulte ou une personne âgée.

Dans un mode de réalisation préféré, au moins un analyte est choisi parmi un antigène ou un anticorps.

Lorsque l'analyte ou un des analytes est un antigène, il s'agit de préférence d'un antigène permettant de diagnostiquer une infection, par exemple une infection causée par un virus, une bactérie, un champignon, un protozoaire ou un métazoaire.

Lorsque l'analyte ou un des analytes est un anticorps, il s'agit de préférence d'un anticorps permettant de diagnostiquer une infection, par exemple une infection causée par un virus, une bactérie, un champignon, un protozoaire ou un métazoaire.

Dans un autre mode de réalisation préféré, au moins un analyte est un acide nucléique.

Dans un autre mode de réalisation préféré, le ou les analytes ne sont pas des acides nucléiques.

Lorsque l'analyte ou un des analytes est un acide nucléique, il s'agit de préférence d'un acide nucléique permettant de diagnostiquer une infection, par exemple une infection causée par un virus, une bactérie, un champignon, un protozoaire ou un métazoaire.

Typiquement, il peut s'agir d'un ou plusieurs antigène(s) et/ou d'un ou plusieurs anticorps et/ou un ou plusieurs acide(s) nucléique(s) spécifique(s) de :
- un virus, tel que VIH (*Virus de l'Immunodéficience Humaine*), en particulier VIH-1 ou VIH-2, VHB (*Virus de l'Hépatite B*), VHC (*Virus de l'Hépatite C*), VPH (*papillomavirus humain*), HTLV (Virus T-lymphotropique humain), en particulier HTLV-I ou HTLV-II,
- un parasite, tel qu'un parasite susceptible de provoquer la Toxoplasmose (en particulier *Toxoplasma gondii*), la Malaria (en particulier un parasite du genre *Plasmodium,* par exemple *Plasmodium falciparum, Plasmodium vivax, Plasmodium ovale, Plasmodium malariae* ou *Plasmodium knowlesi*) ou la maladie de Chagas (en particulier *Trypanosoma cruzi*) chez un homme ou un animal non humain), ou
- une bactérie telle qu'une bactérie susceptible de provoquer la Syphilis (en particulier *Treponema pallidum*) ou la maladie de Lyme (en particulier une bactérie du genre *Borrelia*) chez un homme ou un animal non humain.

Par « parasite », on désigne ici un métazoaire ou un protozoaire parasitant un organisme et entraînant une parasitose. Un parasite au sens de l'invention n'est donc ni un virus, ni une bactérie, ni un champignon.

L'analyte ou au moins un des analytes peut également être un marqueur d'une maladie, tel qu'un marqueur d'une maladie cardiovasculaire ou un marqueur du diabète, un marqueur de l'évolution d'une maladie, telle qu'une hépatite, un marqueur de l'évolution d'une infection causée par un virus, une bactérie, un champignon ou un parasite, un marqueur de résistance à un traitement, par exemple à un traitement antiviral, un traitement antibiotique ou un traitement contre un cancer.

Plusieurs (par exemple deux, trois, quatre, cinq, six, sept, huit, neuf, dix, onze, douze, treize, quatorze, quinze ou plus de quinze) analytes tels que décrits dans la présente demande peuvent être détectés simultanément dans un même échantillon ou dans un même mélange d'échantillons au cours d'un procédé d'analyse multiplexe. Cela peut permettre de diagnostiquer, dans un même échantillon ou dans un même mélange d'échantillons, une ou plusieurs infection(s) ou maladie(s), l'évolution d'une infection ou maladie, une condition (pathologique ou non), un risque de développer une condition (pathologique ou non) ou encore un marqueur de résistance à un traitement chez un sujet.

Les analytes détectés au cours d'un procédé d'analyse multiplexe peuvent être de même nature (par exemple uniquement des anticorps, uniquement des antigènes ou uniquement des acides nucléiques) ou de nature différente (par exemple, au moins un antigène et au moins un anticorps).

### Ligand de capture

Un ligand de capture est une molécule spécifique d'un composé d'intérêt.

Un ligand de capture est, de préférence, spécifique d'un analyte à détecter dans un échantillon. Il s'agit généralement d'un anticorps, d'un antigène, d'un peptide, d'un glucide, d'un lipide ou d'un acide nucléique.

Dans un mode de réalisation avantageux, le ligand de capture n'est pas un acide nucléique. Le ou les ligands de capture sont, par exemple, sélectionnés dans le groupe consistant en un anticorps, un antigène, un peptide, un glucide et un lipide.

Un ligand de capture est fixé à la surface d'un support solide au niveau d'un spot.

Dans un mode de réalisation préféré, le ligand de capture ou un des ligands de capture est un anticorps, un antigène ou un acide nucléique.

Dans un mode de réalisation davantage préféré, le ou les ligands de capture sont un anticorps ou un antigène.

Lorsque le ligand de capture ou un des ligands de capture est un anticorps, il s'agit par exemple d'un anticorps monoclonal ou d'un anticorps polyclonal.

### Ligand de détection

Un ligand de détection est une molécule spécifique d'un composé d'intérêt. Il permet notamment la détection d'un composé d'intérêt fixé à un ligand de capture.

Un ligand de détection d'un analyte est spécifique d'un analyte à détecter dans un échantillon. Il permet notamment la détection d'un analyte fixé à un ligand de capture.

Un ligand de détection peut être un anticorps, un antigène, un peptide, un glucide, un lipide ou un acide nucléique. Il s'agit de préférence d'un anticorps ou un antigène.

Dans un autre mode de réalisation, le ou un des ligands de détection est un acide nucléique.

Dans un autre mode de réalisation, le ligand de détection n'est pas un acide nucléique. Le ou les ligands de détection sont par exemple sélectionnés dans le groupe consistant en un anticorps, un antigène, un peptide, un glucide et un lipide.

Lorsque le ligand de détection ou un des ligands de détection est un anticorps, il s'agit par exemple d'un anticorps monoclonal ou d'un anticorps polyclonal.

Le ligand de détection ou un des ligands de détection est, de préférence, un ligand de détection marqué, c'est-à-dire un ligand de détection auquel est attaché un marqueur de détection (qui peut être par exemple la biotine ou une peroxydase). Le marqueur de détection est attaché (c'est-à-dire couplé) au ligand de détection de manière covalente ou non covalente, de préférence de manière covalente. Le ligand de détection ou un des ligands de détection peut être couplé à un marqueur de détection direct ou indirect.

Lorsque le ligand de détection n'est pas marqué, sa détection peut être réalisée en utilisant un anticorps marqué spécifique dudit ligand de détection.

Un ligand de détection peut être identique au ligand de capture ou à un des ligands de capture utilisés, à l'exception de la présence éventuelle d'un marqueur de détection, et/ou se lier à l'analyte dont il est spécifique au niveau de la même zone que celle liée par le ligand de capture ou un des ligands de capture, en particulier lorsque l'analyte dont il est spécifique est sous forme d'un complexe présentant au moins deux zones de liaison identiques. Ainsi, lorsqu'un des analytes que l'on cherche à détecter est un anticorps, on peut utiliser, en tant que ligand de capture et ligand de détection dudit analyte, un même antigène spécifique dudit anticorps ou deux antigènes différents mais comportant au moins une zone commune (au moins un épitope commun) permettant la reconnaissance par l'anticorps bivalent.

Un ligand de capture et un ligand de détection peuvent également être spécifiques de zones distinctes au niveau d'un analyte.

Dans un mode de réalisation particulier, un ligand de détection et un ligand de capture (par exemple des anticorps) spécifiques d'un même analyte (par exemple un antigène) ne se lient pas au même endroit sur ledit analyte. Par exemple, le ligand de détection peut se lier à une zone dudit analyte dont il est spécifique qui est éloignée de la zone de liaison du ligand de capture, par exemple de manière à éviter une compétition du ligand de capture et du ligand de détection vis-à-vis du composé dont ils sont spécifiques, en raison d'un encombrement stérique.

### Marqueur de détection du ligand de détection

Un marqueur de détection couplé au ligand de détection peut être un marqueur direct ou un marqueur indirect.

Un marqueur direct est un marqueur dont le signal peut être détecté directement, c'est-à-dire sans nécessiter l'ajout préalable d'un rapporteur.

Un marqueur direct est, par exemple, sélectionné dans le groupe consistant en un radio-isotope, un fluorophore, un élément lourd de la classification périodique tel qu'un lanthanide, un composé luminescent, un métal de transition tel que le ruthénium, un chromogène et des nanoparticules colorées, fluorescentes ou luminescentes.

Un composé « luminescent» peut être un composé électroluminescent, un composé thermoluminescent ou un composé chimiluminescent. Dans un mode de réalisation préféré, le composé luminescent est un composé chimiluminescent.

Un exemple de composé luminescent (plus précisément de composé thermoluminescent) que l'on peut utiliser comme marqueur direct consiste en des nanoparticules de silice comprenant (par exemple additionnée de ou dopée par (de l'anglais « doped »)) des molécules d'un composé dioxétane, en particulier le composé 1,2-dioxétane, ou d'un dérivé d'un composé dioxétane, par exemple un dérivé du 1,2-dioxétane.

Un marqueur indirect est un marqueur dont la détection du signal nécessite préalablement l'ajout d'un rapporteur (appelé également premier rapporteur) et, si ledit rapporteur est lui-même couplé à un marqueur de détection indirect, l'ajout d'un deuxième rapporteur du marqueur de détection indirect couplé audit premier rapporteur.

Un rapporteur est un substrat d'un marqueur indirect ou une molécule se liant spécifiquement à un marqueur indirect, ladite molécule étant elle-même un marqueur direct ou indirect ou étant elle-même couplée à un marqueur direct ou indirect.

Un marqueur indirect est, par exemple, sélectionné dans le groupe consistant en une enzyme, un ligand d'un couple ligand-récepteur, un récepteur d'un couple ligand-récepteur, un haptène, un antigène et un anticorps.

Un ligand ou un récepteur d'un couple ligand-récepteur est, par exemple, la biotine, un analogue de la biotine, l'avidine, la streptravidine, la neutravidine ou la digoxigénine.

Un marqueur indirect préféré selon l'invention est une enzyme, de préférence une enzyme produisant un composé luminescent par réaction avec un substrat.

La détection du signal d'un marqueur indirect nécessite au préalable l'ajout d'un rapporteur dudit marqueur indirect.

Un rapporteur d'une enzyme est, par exemple, un substrat de ladite enzyme.

Un rapporteur de la biotine est, par exemple, l'avidine, la streptavidine ou la neutravidine, de préférence couplée à un marqueur direct ou à un marqueur indirect, tel qu'une enzyme ou un catalyseur.

Un rapporteur de la biotine est, de préférence, couplé à un marqueur de détection direct ou indirect, tel qu'une enzyme ou un catalyseur.

Un exemple d'enzyme est la peroxydase, par exemple la peroxydase de raifort (HRP ou POD), la phosphatase alcaline ou la luciferase.

Un rapporteur de la biotine préféré selon l'invention est la streptavidine couplée à une peroxydase, de préférence la peroxydase de raifort. La détection du signal de la biotine nécessite alors l'ajout de la streptavidine couplée à la peroxydase, puis l'ajout du substrat de la peroxydase.

Dans un mode de réalisation préféré, le ligand de détection ou un des ligands de détection est couplé à un marqueur de détection indirect sélectionné parmi une enzyme peroxydase ou une biotine.

Lorsque l'analyte est un acide nucléique, le marqueur de détection ou un des marqueurs de détection peut être détecté en fluorescence, par exemple en utilisant un marquage direct ou indirect avec un fluorophore, en luminescence, de préférence en chimiluminescence, par exemple en utilisant un marquage direct avec un composé luminescent ou un marquage indirect avec une enzyme, notamment une peroxydase, produisant un composé luminescent par réaction avec un substrat.

Lorsque l'analyte est un composé de type protéique, le marqueur de détection ou un des marqueurs de détection est, de préférence, détecté par luminescence, par exemple en utilisant un marquage direct avec un composé de type luminescent, en particulier un composé chimiluminescent ou encore un marquage indirect avec comme rapporteur une enzyme produisant un composé luminescent par réaction avec un substrat.

Dans un mode de réalisation particulier de l'invention, le marqueur de détection ou un des marqueurs de détection utilisés a pour substrat le luminol, l'isoluminol, un composé acridine, coelenterazine, dioxetane ou peroxyoxalic, ou un de leurs dérivés, et en particulier un composé décrit dans la publication Dodeigne C. et al (2000), Talanta 51, 415-439, « Chemiluminescence as diagnostic tool. A review »*.*

Dans un mode de réalisation particulier, le marqueur de détection ou un des marqueurs de détection utilisé a pour substrat le luminol, un analogue du luminol, par exemple l'isoluminol, ou un de leurs dérivés.

Par « luminol », on désigne la 3-aminophthalhydrazide, appelé également 5-amino-2,3-dihydro-phthalazine-1,4-dione. La formule brute du luminol est C₈H₇N₃O₂. A titre d'exemple, on peut utiliser le substrat ELISTAR ETA C Ultra ELISA (Cyanagen, Italie) décrit dans les exemples.

Par « isoluminol », on désigne la 4-aminophthalhydrazide.

Un dérivé du luminol ou d'un analogue du luminol est, de préférence, une molécule obtenue respectivement à partir du luminol ou de l'analogue du luminol, par toute(s) modification(s) possible(s) (par exemple chimique et/ou enzymatique). Un dérivé du luminol ou d'un analogue du luminol est par exemple un substrat d'une enzyme peroxydase, la réaction de ladite enzyme peroxydase avec ledit dérivé du luminol ou de l'analogue du luminol permettant la production d'un composé chimiluminescent.

Un dérivé de l'isoluminol peut être par exemple l'aminoethylisoluminol (ou AEI), l'aminoethylethylisoluminol (ou AEEI), l'aminobutylisoluminol (ou ABI), l'aminobutyléthylisoluminol (ou ABEI), l'aminopentyléthylisoluminol (ou APEI), l'aminohexylisoluminol (ou AHI), l'aminohexyléthylisoluminol (ou AHEI), l'aminooctylmethylisoluminol (ou AOMI) ou l'aminooctylethylisoluminol (ou AOEI), tels que décrits dans la publication Dodeigne C. et al (2000), Talanta 51, 415-439, « Chemiluminescence as diagnostic tool. A review »*.*

Dans un mode de réalisation particulier de l'invention, le « luminol, analogue du luminol (par exemple isoluminol), composé acridine, coelenterazine, dioxetane ou peroxyoxalic, ou un de leurs dérivés », peuvent être apportés sous la forme d'une composition (ou formulation) comprenant ledit luminol, analogue du luminol, composé acridine, coelenterazine, dioxetane ou peroxyoxalic, ou un de leurs dérivés.

### Marqueur de contrôle résistant

La présente invention repose sur l'utilisation d'un ou plusieurs marqueurs de contrôle résistants tels que définis dans les revendications annexées (par exemple un mélange de marqueurs de contrôle résistants) permettant de contrôler la qualité du, de ou des spots présents à la surface d'un support solide, en particulier la présence, localisation et/ou intégrité du, de ou des spots présents à la surface d'un support solide, ce contrôle (ou un des ces contrôles) pouvant être réalisé à la fin du procédé d'analyse mis en œuvre.

Un marqueur de contrôle résistant est un composé pouvant être détecté, par exemple par la production d'un signal. Le signal produit est un signal lumineux. Il peut s'agir également d'un marqueur de contrôle (par exemple un marqueur de contrôle coloré) qui est détecté par absorption de lumière.

Un signal lumineux correspond à l'émission d'une lumière, notamment dans une plage de longueurs d'onde donnée.

Un marqueur de contrôle résistant est un marqueur émettant de la lumière en fluorescence.

Comme son nom l'indique, un marqueur de contrôle résistant selon l'invention est résistant.

Par « marqueur de contrôle résistant », on désigne ici un marqueur dont on détecte un signal à la fin d'un procédé d'analyse. Un marqueur de contrôle résistant est en particulier un marqueur qui reste, en totalité ou au moins partiellement, fixé au niveau d'un spot à la surface d'un support solide, au cours du procédé de préparation du support solide et également au cours du procédé d'analyse mettant en œuvre ledit support, et qui est capable de produire un signal détectable à la fin du procédé d'analyse, c'est-à-dire y compris en présence d'un marqueur de détection d'un ligand de détection d'un analyte et/ou d'un ou plusieurs rapporteurs dans le cas d'un ou plusieurs marqueurs de détection indirects.

Par « capable » dans l'expression « marqueur capable de produire un signal détectable à la fin du procédé d'analyse », on entend que le marqueur de contrôle résistant présent dans un spot produit un signal détectable au niveau dudit spot à la fin du procédé d'analyse, lorsque ledit procédé d'analyse s'est déroulé sans détérioration dudit spot.

Les expressions « pouvant être détecté », « dont on détecte un signal », « capable de produire un signal détectable » ou « produisant un signal détectable » à la fin du procédé d'analyse signifient, en particulier dans le cas où le marqueur de contrôle résistant est un fluorophore, que le « signal détecté » (le signal produit par le marqueur de contrôle résistant et mesuré sur le capteur d'imagerie au niveau de la zone de pixels correspondante) moins (c'est-à-dire auquel on soustrait) le signal moyen des pixels environnants s'élève d'au moins trois écarts-types au-dessus du niveau des signaux des pixels environnants. Lorsque le signal détecté moins le signal moyen des pixels environnants s'élève d'au moins trois écarts-types au-dessus du niveau des signaux des pixels environnants, ledit signal détecté est qualifié de « signal détectable ».

Un marqueur de contrôle selon l'invention est ainsi résistant aux étapes de lavage et de mise en incubation avec les différents réactifs utilisés au cours d'un procédé d'analyse, tels que le ou les ligands de détection, le ou les rapporteurs, le ou les substrats, l'échantillon, un ou des diluants.

En particulier, un marqueur de contrôle résistant selon l'invention est résistant à l'utilisation du ou des marqueurs de détection d'un ligand de détection d'un analyte, le cas échéant au (premier) rapporteur du ou des marqueurs de détection d'un ligand de détection d'un analyte et le cas échéant au (deuxième) rapporteur du marqueur indirect couplé audit premier rapporteur.

Dans un mode de réalisation préféré, un marqueur de contrôle résistant selon l'invention est résistant à l'utilisation d'une ou plusieurs enzymes, en particulier une phosphatase alcaline et/ou une peroxydase, et au(x) substrat(s) de ladite ou desdites enzymes.

Un marqueur de contrôle préféré selon l'invention est résistant à l'utilisation d'un substrat sélectionné dans le groupe consistant en le luminol, l'isoluminol ou un de leurs dérivés.

Dans un mode de réalisation avantageux, un marqueur de contrôle résistant selon l'invention est également résistant à l'utilisation d'une composition (ou formulation) comprenant un substrat sélectionné dans le groupe consistant en le luminol, l'isoluminol ou un de leurs dérivés. Ladite composition peut par exemple comprendre du peroxyde d'hydrogène et/ou des molécules chimiques (ou cofacteurs) contribuant à l'efficacité du luminol, de l'isoluminol ou d'un de leurs dérivés

Par « résistant à l'utilisation d'un composé » ou « résistant à un composé », on entend ici que le marqueur de contrôle est résistant à l'utilisation dudit composé au cours d'un procédé d'analyse. En particulier, le marqueur de contrôle est résistant à sa mise en présence avec ledit composé.

Pour déterminer si un composé donné est un marqueur de contrôle résistant selon l'invention, il est possible de mettre en œuvre le procédé comprenant les étapes suivantes :
a) déposer ledit composé à la surface d'un support solide, pour former au moins un spot,
b) de préférence, saturer la surface du support solide,
c) de préférence, sécher la surface du support solide,
d) éventuellement réaliser une ou plusieurs étapes de lavage,
e) éventuellement mettre le ou les spots en présence d'un ligand de détection,
f) éventuellement réaliser une ou plusieurs étapes de lavage,
g) éventuellement mettre le ou les spots en présence d'au moins un rapporteur ou d'au moins une enzyme, par exemple une enzyme peroxydase couplé ou non à un rapporteur,
h) éventuellement réaliser une ou plusieurs étapes de lavage,
i) mettre le ou les spots en présence d'au moins un substrat (ledit substrat étant de préférence celui qui sera utilisé dans le procédé d'analyse dans lequel on souhaite mettre en œuvre ledit marqueur de contrôle résistant), de préférence un substrat d'une enzyme peroxydase, par exemple le luminol,
j) mesurer le signal produit par ledit composé au niveau du ou des spots, et
k) si le signal mesuré est mesuré à l'étape j) est un signal détectable, conclure que ledit composé est un marqueur de contrôle résistant.

Le « signal détectable » est notamment tel que défini ci-dessus.

Le type de signal mesuré à l'étape j) dépend du composé testé. L'homme du métier sait quel type de signal doit être détecté et comment le détecter, en fonction du composé testé.

Le ou un des ligands de détection de l'étape e) peut être couplé à un marqueur de détection du type biotine, le ou un des rapporteur de l'étape g) peut être un rapporteur du type streptavidine couplé à une enzyme, par exemple une peroxydase, et/ou le ou un des substrats de l'étape i) peut être un substrat de ladite enzyme, par exemple le luminol, un analogue du luminol, et/ou un dérivé du luminol ou d'un analogue du luminol.

De manière surprenante, les inventeurs ont ainsi mis en évidence le caractère résistant de marqueurs de contrôle ayant été simplement déposés à la surface d'un support solide, en mélange avec un composé d'intérêt, par exemple un ligand de capture, afin de former des spots. Ainsi, dans un mode de réalisation particulier de l'invention, un marqueur de contrôle résistant selon l'invention n'est pas fixé de manière covalente à la surface du support solide et/ou n'est pas couplé de manière covalente à un ligand de capture.

Enfin, dans un mode de réalisation préféré de l'invention, un marqueur de contrôle résistant interfère peu ou, de préférence, n'interfère pas avec le signal produit par un marqueur de détection d'un ligand de détection d'un analyte utilisé dans un procédé d'analyse.

Par l'expression « signal produit par le marqueur de détection d'un ligand de détection d'un analyte », on désigne le signal produit par le marqueur de détection du ligand de détection d'un analyte lui-même ou correspondant au marqueur de détection du ligand de détection. En effet, par exemple dans le cas d'une enzyme ou d'un autre type de marquage indirect, le signal n'est pas produit par le marqueur de détection du ligand de détection d'un analyte lui-même, mais par le rapporteur ou un composé produit par la réaction d'un rapporteur, par exemple d'une enzyme, avec son substrat ; dans ce cas, ce signal correspond au marqueur de détection du ligand de détection d'un analyte.

L'expression « un marqueur de contrôle résistant qui interfère peu avec le signal produit par le marqueur de détection d'un ligand de détection d'un analyte » signifie que la fonctionnalité du procédé d'analyse pour l'utilisateur n'est pas affectée par la présence du marqueur résistant, en particulier que la diminution de la sensibilité est inférieure à 40%, de préférence inférieure à 35%, plus préférentiellement inférieure à 30%, plus préférentiellement encore inférieure à 25%. Selon un mode de réalisation particulier, cette expression signifie en outre qu'il n'y a pas de dégradation de la spécificité.

L'expression « il n'y a pas de dégradation de la spécificité » signifie que l'augmentation de la valeur seuil est inférieure ou égale à 40%, de préférence inférieure ou égale à 35%, plus préférablement inférieure ou égale à 30%, et plus préférablement encore inférieure ou égale à 25%.

La valeur seuil est la moyenne des signaux obtenus pour des échantillons négatifs à l'issue du procédé d'analyse additionnée de 12 fois l'écart-type des signaux de ces échantillons.

Un marqueur de contrôle résistant peut être neutre, chargé positivement ou négativement.

Par « molécule chargée positivement ou négativement », on entend, dans la présente demande, une molécule qui comporte une charge globale respectivement positive ou négative, en particulier qui comporte au global une, deux, trois, quatre ou plus de quatre charge(s) respectivement positive(s) ou négative(s).

Un marqueur de contrôle résistant peut comprendre ou être couplé à une molécule porteuse, par exemple une protéine telle que la BSA.

Lorsque le marqueur de contrôle résistant « comprend » une molécule porteuse, généralement il comprend ou consiste en un marqueur de contrôle (par exemple un fluorophore) et une molécule porteuse (par exemple la BSA), ledit marqueur de contrôle étant couplé à ladite molécule porteuse. Ce couplage peut permettre de rendre résistant un marqueur de contrôle qui ne serait pas résistant en absence de couplage.

Le couplage entre un marqueur de contrôle ou un marqueur de contrôle résistant et une molécule porteuse résulte d'une liaison covalente ou non covalente entre le marqueur de contrôle résistant et la molécule porteuse, de préférence d'une liaison covalente.

Une molécule porteuse pouvant être couplée de manière covalente ou non covalente au marqueur de contrôle ou au marqueur de contrôle résistant est, par exemple, sélectionnée dans le groupe consistant en la BSA, une immunoglobuline (appelée également Ig), un dextran, de la poly-lysine et un co-polymère mixte, en particulier un co-polymère d'acides aminés différents (co-polymère lysine-tyrosine par exemple).

Un autre exemple de molécule porteuse pouvant être couplée de manière covalente ou non covalente au marqueur de contrôle ou au marqueur de contrôle résistant est un oligonucléotide ou un polynucléotide, en particulier un ADN ou un ARN.

Un marqueur de contrôle résistant selon l'invention peut être par exemple un marqueur sélectionné à l'issue du procédé de sélection d'un marqueur de contrôle résistant tel que défini ci-dessous.

### Fluorophore de contrôle résistant

Un marqueur de contrôle résistant selon l'invention est un fluorophore. Il est, dans ce cas, appelé fluorophore de contrôle résistant.

Un fluorophore, appelé également fluorochrome ou molécule fluorescente, est une substance, en particulier une substance chimique ou protéique (ou polypeptidique), capable d'émettre de la lumière de fluorescence après excitation avec une énergie lumineuse.

Un fluorophore comprend généralement plusieurs noyaux aromatiques conjugués et/ou des molécules planes et cycliques qui possèdent une ou plusieurs liaisons π.

Un fluorophore peut être une protéine fluorescente, par exemple la B-Phycoerythrine.

Un marqueur de contrôle résistant selon l'invention, en particulier un fluorophore de contrôle résistant est, de préférence caractérisé en ce que son spectre d'excitation ne recouvre pas le spectre d'émission d'un marqueur de détection d'un ligand de détection d'un analyte ou correspondant à un marqueur de détection d'un ligand de détection d'un analyte (utilisé dans un procédé d'analyse) et en ce que son spectre d'émission ne recouvre pas ou recouvre partiellement le spectre d'émission d'un marqueur de détection d'un ligand de détection d'un analyte ou correspondant à un marqueur de détection d'un ligand de détection d'un analyte (utilisé dans un procédé d'analyse).

Le « spectre d'émission ou spectre d'excitation correspondant à un marqueur de détection d'un ligand de détection » est, par exemple, le spectre d'émission ou le spectre d'excitation d'un marqueur direct ou d'un rapporteur d'un marqueur de détection indirect couplé au ligand de détection, d'un rapporteur d'un marqueur de détection indirect couplé à un rapporteur (en particulier d'un marqueur de détection indirect d'un ligand de détection), ou un composé produit par la réaction d'un rapporteur de type enzyme avec son substrat.

Dans le cas d'un fluorophore résistant selon l'invention, le spectre d'excitation peut correspondre au spectre d'absorption.

Un marqueur de contrôle résistant préféré selon l'invention, en particulier un fluorophore de contrôle résistant préféré, est un fluorophore caractérisé en ce que son spectre d'excitation ne recouvre pas le spectre d'émission du luminol, d'un de ses analogues, en particulier de l'isoluminol, et/ou un de leurs dérivés, et en ce que son spectre d'émission ne recouvre pas ou recouvre partiellement le spectre d'émission du luminol, d'un de ses analogues, en particulier de l'isoluminol, et/ou un de leurs dérivés.

Par « spectre d'émission du luminol, d'un de ses analogues, en particulier de l'isoluminol, ou un de leurs dérivés », on désigne le spectre d'émission du composé chimiluminescent résultant de la réaction du luminol, d'un de ses analogues, en particulier de l'isoluminol, ou un de leurs dérivés avec une enzyme peroxydase.

De préférence, un marqueur de contrôle résistant selon l'invention, en particulier un fluorophore de contrôle résistant est caractérisé en ce que son spectre d'émission ne recouvre pas le spectre d'émission du luminol, de l'isoluminol ou un de leurs dérivés.

Lorsque le spectre d'émission d'un marqueur de contrôle résistant selon l'invention, en particulier d'un fluorophore de contrôle résistant, recouvre partiellement le spectre d'émission du marqueur de détection d'un ou plusieurs ligands de détection ou correspondant au marqueur de détection d'un ou plusieurs ligands de détection, il est avantageux d'utiliser un filtre permettant d'éliminer les longueurs d'onde émises par ledit marqueur de détection pour ne conserver que le signal spécifique émis par le fluorophore et/ou un filtre permettant d'éliminer les longueurs d'onde émises par ledit fluorophore, pour ne conserver que le signal spécifique émis par ledit marqueur de détection.

Dans un mode de réalisation préféré, il n'y a pas de transfert d'énergie par résonance entre :
- le ou les marqueurs de contrôle résistants selon l'invention, en particulier le ou les fluorophores de contrôle résistants, et
- le ou les marqueurs de détection d'un ligand de détection et/ou le ou les composés produisant un signal correspondant au(x) marqueur(s) de détection d'un ligand de détection utilisés dans le procédé d'analyse,
en particulier entre le ou les marqueurs de contrôle résistants selon l'invention (en particulier le ou les fluorophores de contrôle résistants) et un composé luminescent obtenu par réaction du luminol, d'un de ses analogues, par exemple l'isoluminol, et/ou d'un de leurs dérivés avec une enzyme peroxydase.

Dans un mode de réalisation avantageux, le ou les marqueurs de contrôle résistants selon l'invention, en particulier le ou les fluorophores de contrôle résistants, ont des plages de longueurs d'onde d'excitation et des plages de longueurs d'onde d'émission qui sont strictement supérieures aux plages de longueurs d'onde d'émission du luminol, d'un de ses analogues, par exemple l'isoluminol, et/ou d'un de leurs dérivés, ou strictement inférieures aux plages de longueurs d'onde d'émission du luminol, d'un de ses analogues, par exemple l'isoluminol, et/ou d'un de leurs dérivés.

A titre d'exemple, lorsque le luminol est utilisé en tant que substrat, un marqueur de contrôle résistant selon l'invention, en particulier un fluorophore de contrôle résistant préféré selon l'invention, n'émet pas de lumière aux environs de 425 nm, en particulier de 375 nm à 550 nm, de préférence de 375 nm à 580 nm, plus préférentiellement de 350 nm à 580 nm. En d'autres termes, lorsque le luminol est utilisé en tant que substrat, un fluorophore de contrôle résistant préféré selon l'invention émet de la lumière en dehors de longueurs d'ondes allant de 375 nm à 550 nm, de 375 nm à 580 nm ou de 350 nm à 580 nm. Il peut par exemple émettre de la lumière uniquement à des longueurs d'ondes inférieures (ou inférieures ou égales) à 375 nm, 370 nm, 360 nm ou 350 nm, ou uniquement à des longueurs d'ondes supérieures (ou supérieures ou égales) à 550 nm, 560 nm, 570 nm, 580 nm, 590 nm ou 600 nm.

L'expression « d'une valeur X à une valeur Y » signifie que les bornes X et Y sont comprises.

Dans un mode de réalisation particulier, de manière surprenante, le pH basique du milieu réactionnel contenant le luminol, un analogue du luminol et/ou un de leurs dérivés, la présence de peroxyde, d'un ou plusieurs agents favorisant le transfert d'électrons et d'un catalyseur d'acylation dans le milieu réactionnel n'entraînent pas de décalage spectral à l'origine d'un problème dans la détection du signal émis par le marqueur de contrôle résistant selon l'invention et en particulier par le fluorophore de contrôle résistant selon l'invention.

Selon un mode de réalisation particulier de l'invention, un marqueur de contrôle résistant selon l'invention, en particulier un fluorophore de contrôle résistant selon l'invention, a une fenêtre d'absorption qui ne comprend pas la plage de longueurs d'onde correspondant au signal émis par le marqueur de détection du ligand de détection et en particulier qui ne comprend pas le plage de longueurs d'onde d'émission du luminol. Par exemple, un fluorophore de contrôle résistant préféré selon l'invention n'absorbe pas les longueurs d'ondes allant de 375 à 530 nm, de 375 à 550 nm, ou de 350 à 580 nm (bornes comprises). Ainsi, on peut par exemple utiliser un fluorophore qui absorbe uniquement des longueurs d'onde inférieures (ou inférieures ou égales) à 375, 370, 360 ou 350 nm, ou un fluorophore qui absorbe uniquement des longueurs d'onde supérieures (ou supérieures ou égales) à 530, 540, 550, 560, 570, 580, 590 ou 600 nm.

Selon un mode de réalisation particulier, un marqueur de contrôle résistant peut être un fluorophore neutre, un fluorophore chargé positivement ou un fluorophore chargé négativement ou une protéine fluorescente, ledit fluorophore étant éventuellement couplé à une molécule porteuse, par exemple une protéine telle que la BSA.

Dans un mode de réalisation préféré, le fluorophore de contrôle résistant comprend au moins un groupe fonctionnel chargé positivement et en particulier au moins une fonction amine.

Selon un mode de réalisation particulier, un marqueur de contrôle résistant comprend ou consiste en au moins un fluorophore et au moins une molécule porteuse (par exemple la BSA), ledit au moins un fluorophore étant couplé à ladite au moins une molécule porteuse, et ledit au moins un fluorophore pouvant être par exemple un fluorophore neutre, chargé positivement ou chargé négativement, ou une protéine fluorescente.

Une molécule porteuse est notamment telle que définie ci-dessus dans le paragraphe « analyte ».

Un fluorophore de contrôle résistant est, par exemple, sélectionné dans le groupe consistant en une coumarine, une rhodamine, une carbopyronine, une oxazine, du benzopyrylium, une phycoérythrine et leurs dérivés. Ledit fluorophore est éventuellement couplé à une molécule porteuse, par exemple une protéine telle que la BSA.

Un fluorophore de contrôle résistant est, par exemple, sélectionné dans le groupe consistant en une coumarine, une rhodamine, une carbopyronine, une oxazine, un dérivé du benzopyrylium, leurs dérivés, et une phycoérythrine. Un fluorophore de contrôle résistant est par exemple choisi parmi les composés décrits dans la demande WO00/64986 A1 et/ou commercialisés par la société Atto-Tec.

Un fluorophore de contrôle résistant préféré est sélectionné dans le groupe consistant en une carbopyronine, une oxazine, un dérivé d'une oxazine, un dérivé du benzopyrylium et une phycoérythrine.

Un autre fluorophore de contrôle résistant préféré est sélectionné dans le groupe consistant en un dérivé de carbopyronine, une oxazine, un dérivé d'une oxazine, un dérivé du benzopyrylium et une phycoérythrine

Un fluorophore de contrôle résistant davantage préféré est sélectionné dans le groupe consistant en une carbopyronine, un dérivé du benzopyrylium et une phycoérythrine.

Un autre fluorophore de contrôle résistant davantage préféré est sélectionné dans le groupe consistant en un dérivé de carbopyronine, un dérivé du benzopyrylium et une phycoérythrine.

A titre d'exemple, on peut citer la carbopyronine Atto 633 commercialisée par la société Atto-Tec et ses dérivés, en particulier un dérivé amine, ainsi que des dérivés de benzopyrylium tel que ceux commercialisés par la société Dyomics, en particulier le Dye 634 ou le Dye 630 lorsqu'ils sont couplés à une molécule porteuse, par exemple la BSA, ou le dérivé amine du Dye 634 ou du Dye 630, couplé ou non à une porteuse, par exemple de la BSA.

Une carbopyronine préférée selon l'invention est une molécule comprenant la structure de base suivante :

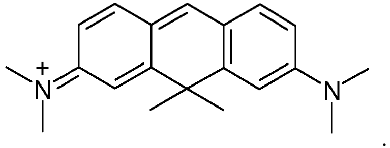

Une carbopyronine pouvant être utilisée comment fluorophore de contrôle résistant, telle que le dérivé amine de l'Atto 633, a par exemple les caractéristiques suivantes : longueur d'onde d'absorption maximale = 629 nm, coefficient d'absorption molaire à la longueur d'onde d'absorption maximale = 1,3 x 10⁵ M⁻¹ cm⁻¹, longueur d'onde d'émission maximale = 657 nm et rendement quantique = 64 %.

Un dérivé du benzopyrylium pouvant être utilisé comment fluorophore de contrôle résistant a par exemple les caractéristiques suivantes :
- longueur d'onde d'absorption maximale (dans l'éthanol) : 635 nm,
- longueur d'onde d'émission maximale (dans l'éthanol) : 658 nm, et
- coefficient d'absorption molaire à la longueur d'onde d'absorption maximale : 200 000 M⁻¹cm⁻¹.

Il s'agit par exemple du fluorophore appelé Dye 634 (sous forme couplée à une molécule porteuse, par exemple la BSA) de formule :

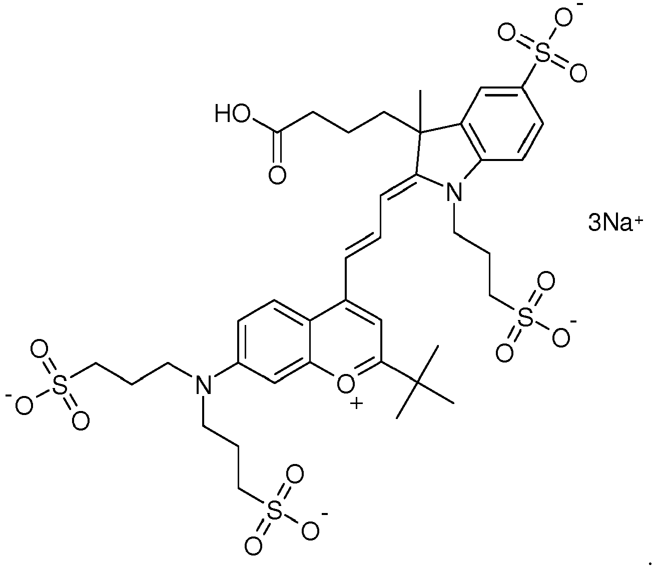

Le Dye 634 peut également être utilisé comment fluorophore de contrôle résistant sous sa forme amine (Dye 634-amine). Il peut être utilisé couplé ou non à molécule porteuse et en particulier à de la BSA.

Encore un autre dérivé du benzopyrylium pouvant être utilisé comment fluorophore de contrôle résistant a par exemple les caractéristiques suivantes :
- longueur d'onde d'absorption maximale (dans l'éthanol) : 636 nm,
- longueur d'onde d'émission maximale (dans l'éthanol) : 657 nm, et
- coefficient d'absorption molaire à la longueur d'onde d'absorption maximale : 200 000 M⁻¹cm⁻¹.

Il s'agit par exemple du Dye 630 (sous forme couplée à une molécule porteuse, par exemple la BSA).

Le Dye 630 a la formule suivante :

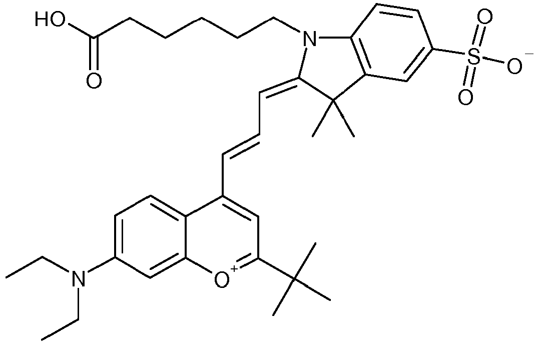

Le Dye 630 peut également être utilisé sous sa forme amine (Dye 630-amine), couplé ou non couplé à une molécule porteuse, par exemple la BSA.

### Enzyme en tant que marqueur de contrôle résistant (ne faisant pas partie de l'invention)

Un autre exemple de marqueur de contrôle résistant décrit ici est une enzyme produisant par exemple un composé luminescent par réaction avec un substrat de ladite enzyme,

De préférence, le composé luminescent est un composé chimiluminescent.

Une enzyme pouvant être utilisée comme marqueur de contrôle résistant est par exemple une phosphatase alcaline ou une luciférase.

Un substrat de la phosphatase alcaline est par exemple le substrat Lumi-Phos 530 ou Lumi-Phos plus commercialisé par Lumigen.

Un substrat de la luciferase est par exemple le substrat luciferine ou coelenterazine.

Lorsque le marqueur de contrôle résistant est une enzyme produisant un composé luminescent, le signal détecté correspondant au marqueur de contrôle résistant est le signal émis par le composé luminescent produit par réaction de ladite enzyme avec un substrat de ladite enzyme.

### Détection du signal

Le signal produit par le(s) marqueur(s) de détection du ligand de détection ou produit par le(s) marqueur de contrôle(s) résistant(s) est détecté de manière directe ou indirecte.

Selon le type de marqueur utilisé, le signal peut par exemple être détecté en fluorescence ou en luminescence, en particulier en chimiluminescence.

Le signal émis par un marqueur de type fluorophore peut être lu directement en fluorescence.

Un marqueur de type enzyme nécessite l'ajout d'un substrat permettant la production d'un produit détectable, par exemple l'ajout d'un substrat permettant la production de lumière.

Un marqueur de contrôle résistant émettant de la lumière en électroluminescence nécessite l'ajout d'un complexe de Ruthénium et l'ajout de Tripropylamine (TPA), ainsi que l'application d'un courant électrique permettant la production de lumière.

Comme indiqué ci-dessus, un marqueur indirect de type biotine nécessite l'ajout d'un rapporteur, de préférence d'un rapporteur couplé à un marqueur de détection direct ou indirect.

Si le rapporteur est couplé à un marqueur indirect de type enzyme, par exemple la peroxydase, il est nécessaire d'ajouter dans une étape ultérieure le substrat de cette enzyme, par exemple le luminol ou un analogue du luminol, tel que l'isoluminol ou un dérivé du luminol ou d'un analogue du luminol.

Le signal peut avantageusement être détecté au moyen d'un dispositif selon l'invention, tel que défini ci-après.

Dans un mode de réalisation préféré, le signal émis par le(s) marqueur(s) de contrôle résistant(s) est détecté en fluorescence et le signal émis par le(s) marqueur(s) de détection du ligand de détection est émis en luminescence, par exemple en chimiluminescence.

Généralement, la réaction de chimiluminescence est effectuée au moyen d'un kit comprenant au moins deux solutions.

La première solution comprend le substrat de la peroxydase, par exemple le luminol, l'isoluminol et/ou un dérivé du luminol ou de l'isoluminol, et un médiateur d'électrons ; la deuxième solution comprend un oxydant. A titre d'exemple, il est possible d'utiliser le kit « Immun-star western C » (Bio-Rad, Etats-Unis), « ELISTAR ETA C Ultra ELISA » (Cyanagen, Italie), « Supersignal West Pico » (Thermo Scientific, Etats-Unis), « Chemiluminescent Sensitive Plus HRP» (Surmodics, Etats-Unis).

### Support solide pour une détection améliorée d'au moins un analyte dans un échantillon

Le ou les supports utilisés pour la mise en œuvre du procédé d'analyse selon l'invention sont des supports solides.

Selon un mode de réalisation particulier, un support solide est obtenu par le procédé de préparation d'un support solide selon l'invention.

Un support solide peut être en toute matière appropriée pour la mise en œuvre du procédé d'analyse. Un support solide est par exemple un support à base d'un polymère ou d'un mélange de polymères.

Un support solide approprié est, par exemple, un support en polystyrène, polypropylène, poly(méth)acrylate, polybutadiène ou leurs combinaisons.

Un autre exemple de support solide approprié est une membrane, par exemple une membrane en nitrocellulose, PVDF (fluorure de polyvinylidène), nylon ou leurs combinaisons.

Encore un autre exemple de support solide approprié est un support inorganique, par exemple une lame de verre et/ou un support métallique.

Un support solide préféré est en polystyrène ou polypropylène.

Un support solide comprend au moins un compartiment (appelé également zone d'analyse), de préférence au moins deux compartiments.

Selon un mode de réalisation particulier de l'invention, un support solide comprend un unique compartiment. Ledit compartiment unique peut être un compartiment comportant une ou plusieurs parois. Alternativement, ledit compartiment unique peut être dépourvu de parois et s'assimiler alors au support solide lui-même. Le fond du compartiment peut alors consister en la face supérieure du support solide. Un exemple d'un tel support solide comprenant un unique compartiment comportant ou non une ou plusieurs parois est une lame ou une membrane.

Selon un mode de réalisation particulier de l'invention, le support solide, qui peut être par exemple une microplaque ou une membrane, comprend au moins deux compartiments.

Lorsque le support solide comprend au moins deux compartiments, ils sont isolés les uns des autres, de sorte qu'ils ne communiquent pas entre eux c'est-à-dire de sorte que les différentes compositions ou solutions utilisées pour l'analyse ne puissent pas circuler d'un compartiment à un autre pendant l'analyse. Ainsi, une solution ajoutée dans un compartiment ne va pas dans les autres compartiments. Par exemple, le ou les compartiments comprennent ou sont constitués d'un fond et d'une ou plusieurs parois, la ou lesdites parois isolant le ou les compartiments les uns des autres de sorte qu'ils ne communiquent pas entre eux.

Typiquement, au moins un (par exemple un ou deux) compartiment du support solide est utilisé par échantillon à analyser.

Dans un mode de réalisation particulier de l'invention où le support solide (par exemple une lame ou d'une membrane) comprend un unique compartiment, typiquement, au moins un (par exemple un ou deux) support solide est utilisé par échantillon à analyser.

Le support solide est par exemple une microplaque. Dans ce cas, un exemple de compartiment est un puits.

Une microplaque est typiquement une microplaque de 96 puits ou de 384 puits.

La présente invention a ainsi pour objet un support solide pour la détection d'au moins un analyte dans au moins un échantillon, caractérisé en ce que ledit support solide comprend au moins un compartiment comprenant au moins un spot, ledit spot comprenant au moins un marqueur de contrôle résistant et au moins un ligand de capture.

Un marqueur de contrôle résistant est notamment tel que défini ci-dessus, en particulier dans les paragraphes « marqueur de contrôle résistant », « fluorophore de contrôle résistant » et « enzyme en tant que marqueur de contrôle résistant » ou encore tel qu'obtenu par le procédé de sélection défini ci-dessous dans le paragraphe « procédé de sélection d'un marqueur de contrôle résistant ».

En particulier, ledit ou lesdits marqueurs de contrôle résistants sont des marqueurs qui restent au moins partiellement fixés au niveau dudit spot à la surface du support solide au cours de la mise en œuvre d'un procédé de détection d'au moins un analyte, de sorte à produire un signal détectable à l'issue dudit procédé de détection.

Un compartiment du support solide destiné à analyser un échantillon comprend au moins un spot, par exemple deux spots, trois spots, quatre spots ou cinq spots, ou au moins six spots, de préférence six spots, sept spots, huit spots, plus préférentiellement au moins neuf spots, par exemple neuf spots, dix spots, onze spots, douze spots, treize spots, quatorze spots, quinze spots, seize spots ou plus de seize spots.

Par « spot », on entend ici une zone à la surface d'un compartiment d'un support solide comprenant au moins un marqueur de contrôle résistant et/ou au moins un composé d'intérêt, de préférence au moins un marqueur de contrôle résistant et au moins un composé d'intérêt, par exemple un ligand de capture. Le(s) marqueur(s) de contrôle résistant(s) et le(s) composé(s) d'intérêt se fixent ainsi en même temps à ladite zone à la surface du compartiment, par des interactions physico-chimiques non covalentes (en particulier de type liaisons faibles, par exemple, ioniques, van der Waals, hydrogène et/ou hydrophobe) et/ou par des liaisons covalentes.

Un spot peut comprendre, outre le ou les composé(s) d'intérêt, au moins un polymère, en particulier au moins un polymère comportant des groupes hydrophiles, par exemple au moins un hydrogel.

Dans un mode de réalisation particulier, tous les spots d'un compartiment, de préférence tous les spots d'un support solide, comprennent un unique marqueur de contrôle résistant ou un unique mélange de marqueurs de contrôle résistants qui peut être utilisé à une même concentration dans tous les spots ou à des concentrations différentes.

Alternativement, différents (au moins deux) marqueurs de contrôle résistants et/ou différents (au moins deux) mélanges de marqueurs de contrôle résistants peuvent être utilisés dans les spots d'un même compartiment d'un support solide.

La présente invention a particulièrement pour objet un support solide pour la détection d'au moins un analyte dans au moins un échantillon, caractérisé en ce que ledit support solide comprend au moins un compartiment comprenant au moins un spot, ledit spot comprenant un seul marqueur de contrôle résistant et au moins un ligand de capture.

De préférence, tous les spots destinés à la détection d'un analyte, en particulier tous les spots comprenant un ligand de capture, ne comprennent qu'un seul marqueur de contrôle résistant qui est, de préférence, le même dans tous les spots destinés à la détection d'un analyte d'un même compartiment et, plus préférentiellement, qui est le même dans tous les spots destinés à la détection d'un analyte de tous les compartiments d'un support solide.

Un spot correspond à une zone bien délimitée, par exemple comprise de 0,0078 mm² à 5,309 mm², de préférence de 0,196 mm² à 3,142 mm², plus préférentiellement comprise de 0,503mm² à 2,011 mm².

Un spot peut être de forme discoïdale, cylindrique ou hémisphérique ou approximativement de forme discoïdale, cylindrique ou hémisphérique, par exemple ovale, en particulier lorsque le support solide est une microplaque ou une lame.

Alternativement, un spot peut être de forme carrée ou rectangulaire (en particulier une bande), par exemple lorsque le support solide est une membrane, ou de tout autre forme.

Les spots sont obtenus par des techniques bien connues de l'homme du métier, telles que celles divulguées dans les brevets US 7 470 547 B2, US 6 576 295 B2, US 5 916 524 A et US 5 743960 A.

Par exemple, un spot est obtenu par le dépôt d'au moins une goutte d'une solution contenant une quantité déterminée d'au moins un marqueur de contrôle résistant et d'au moins un composé d'intérêt à un endroit précis à la surface du compartiment du support solide.

Lorsqu'un spot comprend au moins un polymère (par exemple au moins un hydrogel), ledit spot peut être obtenu par le dépôt d'au moins une goutte d'une solution contenant une quantité déterminée d'au moins un marqueur de contrôle résistant et d'au moins un composé d'intérêt à un endroit précis à la surface du compartiment sur laquelle ledit polymère a été déposé au préalable.

La surface d'un compartiment est également appelée « phase solide ».

Un composé d'intérêt est généralement un ligand de capture, en particulier un ligand de capture d'un analyte. Un ou plusieurs spots d'un compartiment peuvent servir de spot contrôle et comprendre ainsi un ligand de capture qui n'est pas destiné à détecter un analyte de l'échantillon, ou encore comprendre un autre composé d'intérêt, ou encore ne comprendre aucun composé d'intérêt.

La présente invention a particulièrement pour objet un support solide pour une détection d'au moins un analyte dans un échantillon, caractérisé en ce que ledit support solide comprend au moins deux compartiments comprenant au moins un spot, ledit spot comprenant au moins un marqueur de contrôle résistant et au moins un ligand de capture.

Dans un mode de réalisation particulier, une partie ou la totalité des compartiments d'un support solide ont la même composition en spots.

Dans un autre mode de réalisation particulier, une partie ou la totalité d'un support solide ou des compartiments d'un support solide comprend ou consiste en plusieurs (par exemple deux) groupes distincts (ou types) de spots ou de compartiments, chacun des groupes distincts ayant une composition en spots différente (de par le nombre de spots et/ou de par le(s) marqueur(s) de contrôle résistant(s) et/ou le(s) ligand(s) de capture et/ou le(s) composé(s) d'intérêt présents dans les spots de chaque groupe).

### Préparation d'un support d'analyse comprenant un marqueur de contrôle résistant

La présente invention a également pour objet un procédé de préparation d'un support solide pour la détection d'au moins un analyte dans au moins un échantillon comprenant les étapes suivantes :
a) déposer, à la surface d'au moins un compartiment, de préférence au moins deux compartiments, d'un support solide, un mélange comprenant au moins un marqueur de contrôle résistant et au moins un ligand de capture, pour obtenir un spot,
b) répéter l'étape a) n-1 fois, n étant un nombre entier supérieur ou égal à 1, pour obtenir n spots destinés à la détection d'un analyte à la surface dudit ou desdits compartiments,
c) éventuellement saturer la surface dudit ou desdits compartiments, et
d) éventuellement sécher la surface dudit ou desdits compartiments.

Le support solide, le(s) marqueur(s) de contrôle résistant(s) et le(s) ligand(s) de capture sont notamment tels que définis ci-dessus.

Dans l'étape a), un mélange comprenant au moins un marqueur de contrôle résistant et au moins un ligand de capture est déposé à la surface d'au moins un compartiment d'un support solide, de préférence au moins deux compartiments d'un support solide, pour obtenir un spot.

Dans l'étape a), ledit ou lesdits marqueurs de contrôle résistants sont des marqueurs qui restent au moins partiellement fixés au niveau dudit spot à la surface du support solide au cours de la mise en œuvre d'un procédé de détection d'au moins un analyte, de sorte à produire un signal détectable à l'issue dudit procédé de détection.

Un marqueur de contrôle résistant est notamment tel que défini dans les revendications annexées.

Ledit ou lesdits marqueurs de contrôle résistant sont des fluorophores, par exemple un mélange de fluorophores. Le ou les fluorophores peuvent être des molécules chimiques fluorescentes ou des protéines fluorescentes.

Ledit ou lesdits ligands de capture sont, de préférence, sélectionnés dans le groupe consistant en un anticorps, un antigène, un acide nucléique et leurs combinaisons.

Dans un autre mode de réalisation préféré, ledit ou lesdits ligands de capture ne sont pas des acides nucléiques.

Par exemple, ledit ou lesdits ligands de capture sont sélectionnés dans le groupe consistant en un anticorps et un antigène.

Le mélange est, de préférence, une solution.

Un marqueur de contrôle résistant est présent dans le mélange à une concentration qui n'interfère pas avec la fixation du ou des ligand(s) de capture à la surface du compartiment et qui permet une détection du signal produit par ledit marqueur de contrôle résistant à la fin du procédé d'analyse.

A titre d'exemple, le mélange comprend 0,1 à 100 µg/ml d'un ligand de capture et 0,01 à 100 µg/ml d'un marqueur de contrôle, de préférence dans une solution tampon, par exemple une solution de TBS (Tampon Tris Salin).

La concentration d'un marqueur de contrôle résistant dans le mélange peut par exemple être déterminée par le procédé comprenant les étapes suivantes :
- déposer à la surface d'un support solide au moins deux solutions, chaque solution comprenant ledit marqueur de contrôle résistant et un ligand de capture, la concentration du marqueur de contrôle résistant étant croissante d'une solution à l'autre et la concentration du marqueur de contrôle résistant la plus basse étant supérieure ou égale à une concentration minimale prédéfinie,
- éventuellement saturer la surface du support solide, c'est-à-dire mettre la surface du support solide en présence d'un agent permettant d'éviter les liaisons non spécifiques au support solide,
- éventuellement sécher la surface du support solide,
- mettre en présence des spots un échantillon comportant un analyte dont le ligand de capture est spécifique,
- mettre en présence des spots un ligand de détection spécifique dudit analyte, ledit ligand de détection étant couplé à un marqueur de détection direct ou indirect,
- lorsque ledit ligand de détection est couplé à un marqueur de détection indirect, ajouter un rapporteur (appelé également premier rapporteur) dudit marqueur de détection indirect et lorsque ledit (premier) rapporteur est couplé à un marqueur de détection indirect, ajouter un deuxième rapporteur du marqueur de détection indirect couplé audit (premier) rapporteur du marqueur de détection indirect du ligand de détection,
- détecter un signal produit par le marqueur de contrôle résistant,
- détecter un signal produit par le marqueur de détection du ligand de détection, et
- sélectionner une concentration en marqueur de contrôle résistant qui permet de détecter à la fois le signal produit par le marqueur de contrôle résistant et le signal produit par le marqueur de détection du ligand de détection.

La concentration en marqueur de contrôle résistant permet, de préférence, de détecter à la fois le signal produit par le marqueur de contrôle résistant et le signal produit par le marqueur de détection du ligand de détection, sans perte de sensibilité significative par rapport à un spot ne comprenant pas le marqueur de contrôle résistant, c'est-à-dire que la fonctionnalité du procédé d'analyse pour l'utilisateur n'est pas affectée par la présence du marqueur résistant, en particulier la diminution de la sensibilité est inférieure à 40%, de préférence inférieure à 35%, plus préférentiellement inférieure à 30%, plus préférentiellement encore inférieure à 25%.

Selon un mode de réalisation particulier, la concentration en marqueur de contrôle résistant permet en outre de détecter le signal produit par le marqueur de contrôle résistant et le signal produit par le marqueur de détection du ligand de détection sans dégradation de la spécificité.

L'expression « il n'y a pas de dégradation de la spécificité » signifie que l'augmentation de la valeur seuil est inférieure ou égale à 40%, de préférence inférieure ou égale à 35%, plus préférablement inférieure ou égale à 30%, et plus préférablement encore inférieure ou égale à 25%.

La valeur seuil est la moyenne des signaux obtenus pour des échantillons négatifs à l'issue du procédé d'analyse additionnée de 12 fois l'écart-type des signaux de ces échantillons.

Lorsqu'un ligand de détection est couplé à un marqueur de détection indirect, un rapporteur dudit marqueur de détection indirect est donc ajouté. Lorsque ledit rapporteur (appelé premier rapporteur) est lui-même couplé à un marqueur détection indirect, par exemple une enzyme, un deuxième rapporteur, par exemple un substrat, du marqueur de détection indirect couplé audit premier rapporteur est ajouté.

La concentration minimale en marqueur de contrôle résistant prédéfinie peut, par exemple, être déterminée par la méthode suivante :
- déposer à la surface d'un support solide au moins deux solutions, chaque solution comprenant un marqueur de contrôle résistant et ne comprenant pas de ligand de capture, la concentration du marqueur de contrôle résistant étant croissante d'une solution à l'autre, pour former au moins deux spots,
- éventuellement saturer la surface du support solide, c'est-à-dire mettre la surface du support solide en présence d'un agent permettant d'éviter les liaisons non spécifiques au support solide,
- éventuellement sécher la surface du support solide,
- éventuellement réaliser au moins une des étapes suivantes :
   (i) réaliser une ou plusieurs étapes de lavage,
   (ii) mettre le ou les spots en présence d'un ligand de détection,
   (iii) mettre le ou les spots en présence d'un rapporteur,
   (iv) mettre le ou les spots en présence d'un substrat,
- détecter le signal produit par le marqueur de contrôle résistant, et
- sélectionner la concentration minimale en marqueur de contrôle résistant qui permet la détection d'un signal.

Le dépôt du mélange (ou de la solution) à la surface du support solide ou d'un compartiment du support solide peut être réalisé manuellement, mais de préférence de manière automatisée par un dispositif approprié.

Ainsi, comme indiqué précédemment, un marqueur de contrôle résistant se fixe sur la phase solide du support d'analyse en même temps que le(s) ligand de capture présent(s) dans le mélange (ou la solution).

Dans un mode de réalisation particulier de l'invention, chaque spot d'un compartiment du support solide destiné à la détection d'au moins un analyte comprend au moins un ligand de capture différent, de préférence destiné à détecter un analyte par spot. Toutefois, plusieurs spots d'un compartiment peuvent comprendre au moins un même ligand de capture.

Dans un mode de réalisation particulier de l'invention, un même spot peut comprendre plusieurs ligands de capture différents (par exemple plusieurs anticorps et/ou antigènes), qui sont généralement spécifiques d'une même pathologie, infection ou maladie à détecter (en particulier spécifiques d'un même virus, d'une même bactérie, d'un même champignon ou d'un même parasite), à l'évolution d'une infection ou maladie, à une condition (pathologique ou non) d'un sujet, à un risque de développer une condition (pathologique ou non) ou encore à un marqueur de résistance à un traitement.

Plusieurs spots ou la totalité des spots d'un compartiment peuvent comprendre des ligands de capture destinés à la détection d'un même analyte ; il s'agit par exemple de ligands de capture différents spécifiques d'un même analyte ou encore d'un même ligand de capture présent à différentes concentrations dans les spots.

Dans un mode de réalisation particulier, le marqueur de contrôle résistant ou le mélange de marqueurs de contrôle résistants est le même dans tous les spots, et il peut être ajouté ou non à une même concentration dans chaque spot.

Alternativement, différents marqueurs de contrôle résistants (au moins deux) ou différents mélanges d'au moins deux marqueurs de contrôle résistants peuvent être utilisés dans différents spots d'un compartiment.

Un compartiment peut également comprendre un ou plusieurs spots sans marqueur de contrôle résistant. Toutefois, de préférence, tous les spots dudit compartiment comprennent un marqueur de contrôle résistant.

Un compartiment peut également comprendre un ou plusieurs spots sans ligand de capture, mais de préférence comprenant un autre composé d'intérêt.

Lorsqu'un compartiment comprend un ou plusieurs spots sans ligand de capture, le ou lesdits spots comprennent, de préférence, un marqueur de contrôle résistant.

Dans un mode de réalisation particulier de l'invention, tous les compartiments du support ont la même composition en spots.

Dans un autre mode de réalisation particulier de l'invention, une partie ou la totalité des compartiments d'un support solide comprend ou consiste en plusieurs (par exemple deux) groupes distincts de compartiments, chacun des groupes distincts ayant une composition en spots différente.

Les étapes a) et b) sont réalisées dans une partie ou dans tous les compartiments du support solide, de préférence, dans tous les compartiments du support solide.

Le mélange déposé à la surface d'au moins un compartiment du support solide est mis en incubation par exemple pendant quelques secondes à quelques heures, par exemple à une température comprise de 4°C à 40°C.

Le procédé comprend une éventuelle étape c) de saturation de la surface dudit ou desdits compartiment du support solide, c'est-à-dire consistant à mettre la surface du support solide en présence d'un agent permettant d'éviter les liaisons non spécifiques au support solide. L'étape de saturation a notamment pour objet d'empêcher la fixation non spécifique de composés, lors de la mise en œuvre du procédé d'analyse.

L'agent permettant d'éviter les liaisons non spécifiques au support solide est par exemple une solution de saturation bien connue de l'homme du métier.

Dans l'éventuelle étape d), la surface du ou desdits compartiments est séchée.

Le séchage est, par exemple, réalisé à 56°C ou 37°C ou à température ambiante.

Dans un mode de réalisation particulier, l'étape a), l'étape b) et/ou l'étape c) sont suivies d'une ou plusieurs étapes de lavage.

La présente invention a également pour objet un procédé de préparation d'un support solide tel que défini ci-dessus, comprenant une étape ultérieure e) consistant à contrôler la qualité des spots.

Si le contrôle de la qualité d'un spot est positif, ledit spot peut être utilisé dans le cadre d'un procédé d'analyse.

Si le contrôle de la qualité d'un spot est négatif, ledit spot ne peut pas être utilisé dans le cadre d'un procédé d'analyse, éventuellement de même que le compartiment contenant ledit spot.

La présente divulgation décrit également un kit (ou trousse) caractérisé en ce qu'il comprend ou consiste en au moins un support solide selon l'invention ou obtenu par le procédé de préparation selon l'invention et, le cas échéant, au moins une composition ou solution à utiliser pour mettre en œuvre un procédé d'analyse selon l'invention et/ou une notice d'utilisation.

### Dispositif pour une détection améliorée (ou dispositif pour une double détection)

La présente invention a également pour objet de fournir un dispositif pour la détection d'au moins un analyte dans un échantillon, ledit dispositif comprenant :
- des moyens pour détecter un premier signal et un deuxième signal produits au niveau d'un support solide, et
- des moyens pour définir une grille de lecture à partir de la localisation dudit premier signal et pour lire ledit deuxième signal au niveau de ladite grille de lecture.

Selon un mode de réalisation particulier, cette lecture correspond à une quantification du deuxième signal au niveau des régions d'intérêt.

La détection du premier signal peut se faire avant, après ou simultanément à la détection du deuxième signal.

Le support solide est notamment tel que défini ci-dessus.

Le premier signal peut être un signal en fluorescence ou luminescence, par exemple en chimiluminescence.

Le deuxième signal peut être un signal en fluorescence ou luminescence, par exemple en chimiluminescence.

Dans un mode de réalisation préféré, le premier signal est un signal en fluorescence et le deuxième signal est un signal en luminescence, de préférence en chimiluminescence (ou inversement).

Les moyens pour détecter un premier signal et un deuxième signal produits au niveau d'un support solide sont appelés banc optique.

Un banc optique peut par exemple comprendre ou être constitué de :
- un système d'éclairage,
- un objectif télécentrique,
- une roue filtre, ledit objectif télécentrique étant de préférence couplé sur sa lentille de sortie à ladite roue filtre, et
- une caméra.

Le système d'éclairage est de préférence un système d'éclairage pilotable.

Le système d'éclairage permet d'illuminer le support solide avec plus ou moins d'intensité, par exemple pour révéler la fluorescence ou la géométrie du support solide utilisé.

L'objectif télécentrique est de préférence de grande dimension. En particulier, l'objectif télécentrique couvre la totalité du support solide.

L'objectif télécentrique permet d'imager l'ensemble de la surface à mesurer sans déformer l'image, d'éliminer l'erreur de parallaxe inhérente aux objectifs standards et de garantir une homogénéité de signal sur toute la surface visualisée.

L'objectif télécentrique est, de préférence, couplé sur sa lentille de sortie à une roue filtre.

La roue filtre permet de présenter différents filtres entre l'objectif télécentrique et la caméra (appelée également caméra de prise d'image). Dans un mode de réalisation préféré, il est ainsi possible de sélectionner les longueurs d'ondes atteignant le capteur de la caméra pour pouvoir, dans un cas, séparer les signaux d'excitation des éclairages de ceux émis par au moins un marqueur de contrôle résistant (en particulier au moins un fluorophore) par la présence d'un filtre et, dans l'autre cas, de collecter le maximum de signal produit par au moins un marqueur de détection d'un ligand de détection d'un analyte par la présence d'une fenêtre neutre ne filtrant pas de signal mais garantissant le maintien des propriétés optiques de l'ensemble.

La caméra permet d'effectuer une prise d'image avec des temps d'exposition variables.

Le banc optique ainsi défini permet de prendre une succession d'images du même support, avec des paramètres d'éclairage, filtrage et/ou prise d'images différents, tels que :
- une image dans le visible (appelée également image de positionnement), par exemple en utilisant l'éclairage du système d'éclairage, mais pas de filtre spécifique,
- une image en fluorescence (appelée également image de détection) en utilisant l'éclairage du système d'éclairage et un filtre spécifique de la fluorescence recherchée,
- une image sans éclairage et sans filtre spécifique (appelée également image d'analyse), par exemple pour révéler un signal en chimiluminescence,

Les moyens pour définir une grille de lecture à partir de la localisation dudit premier signal et pour lire le deuxième signal au niveau de ladite grille de lecture peuvent comprendre ou être constitués d'un système d'imagerie.

Dans un mode de réalisation avantageux, le banc optique est donc associé à un système d'imagerie permettant de mettre en œuvre un procédé d'analyse comprenant plusieurs étapes qui garantissent notamment la justesse et la robustesse de l'analyse effectuée, lesdites étapes comprenant :
1. recherche et positionnement du support solide et du ou des compartiments du support solide, en particulier grâce à l'image de positionnement ; ce traitement a pour objectif de réduire toute erreur de positionnement mécanique du support solide et permet d'utiliser une mécanique n'ayant pas une haute précision de positionnement ;
2. à partir de la position du ou des compartiments déterminés à l'étape 1, positionnement dans chaque compartiment d'une grille théorique représentant la position théorique de chacun des spots ; ce positionnement peut être fait à partir d'une grille de référence, par exemple décrite dans le système d'analyse, ladite grille de référence indiquant les coordonnées de chacun des spots par rapport à un point de référence du compartiment ; le point de référence du compartiment est, par exemple, le centre du compartiment ;
3. recherche, à l'aide de l'image de détection réalisée avec l'éclairage et le filtre de fluorescence de tous les événements fluorescents existant dans le ou les compartiments du support solide, et éventuellement, le rejet de certains événements à l'aide d'une sélection des événements par leur taille et leur forme de manière à éliminer ceux qui sont totalement en dehors des spécifications attendues ;
4. comparaison entre :
   ∘ la position des événements précédemment détectés et considérés comme valides,
   ∘ la position théorique des spots attendus telle que définie dans l'étape 2 ;
5. pour chacun des spots attendus, association avec l'événement le plus proche détecté et étant en intersection avec la surface du spot théorique de manière à former des couples « spot théorique »/ « événement fluorescent » permettant de détecter les spots manquants. Pour chaque spot théorique, un événement fluorescent distinct et unique doit exister ;
6. caractérisation de chacun des spots détectés par des critères de diamètre, de forme, de distance par rapport à la position théorique permettant de garantir l'intégrité des spots détectés ;
7. pour chacun des spots détectés, définition de la région d'intérêt correspondante, appelée grille de lecture, notamment définie par la surface et les coordonnées du spot détecté ; et
8. lecture du deuxième signal sur la grille de lecture définie à l'étape 7, en particulier quantification du deuxième signal sur l'image d'analyse au niveau de cette région d'intérêt, c'est-à-dire de la grille de lecture.

### Procédé d'analyse avec contrôle de la qualité des spots

Le procédé d'analyse selon l'invention permet la détection d'au moins un analyte dans au moins un échantillon.

La présente invention a particulièrement pour objet un procédé de détection d'au moins un analyte dans au moins un échantillon comprenant les étapes suivantes :
a) mettre un échantillon à analyser en présence du ou des spots d'un compartiment d'un support solide, ledit spot ou au moins un desdits spots comprenant au moins un marqueur de contrôle résistant et au moins un ligand de capture d'un analyte,
b) mettre en présence du ou des spots dudit compartiment au moins un ligand de détection d'un analyte, ledit ligand de détection d'un analyte étant couplé à un marqueur de détection direct ou indirect,
c) lorsqu'au moins un ligand de détection d'un analyte est couplé à un marqueur de détection indirect, mettre en présence du ou des spots dudit compartiment un rapporteur dudit marqueur de détection indirect,
d) lorsque le rapporteur utilisé à l'étape c) est couplé à un marqueur de détection indirect, mettre en présence du ou des spots dudit compartiment un rapporteur du marqueur de détection indirect couplé audit rapporteur utilisé à l'étape c),
e) détecter un signal produit par au moins un marqueur de contrôle résistant dans ledit compartiment,
f) définir une grille de lecture à partir de la localisation du signal détecté à l'étape e),
g) éventuellement, détecter un signal produit par au moins un marqueur de détection d'un ligand de détection d'un analyte, et
h) éventuellement, lire le signal détecté à l'étape g) sur la grille de lecture définie à l'étape f).

Comme défini ci-dessus, le ou lesdits marqueurs de contrôle résistants sont capables de produire un signal détectable à l'issue d'un procédé d'analyse, c'est-à-dire qu'ils produisent un signal détectable au niveau d'un spot lorsque le procédé d'analyse s'est déroulé sans détérioration dudit spot. En particulier, le ou lesdits marqueurs de contrôle résistants sont capables de produire un signal détectable lors de l'étape e) du procédé ci-dessus.

La présente invention a particulièrement pour objet un procédé de détection d'au moins un analyte dans au moins un échantillon comprenant les étapes suivantes :
a) mettre un échantillon à analyser en présence du ou des spots d'un compartiment d'un support solide, ledit spot ou au moins un desdits spots comprenant au moins un marqueur de contrôle résistant et au moins un ligand de capture d'un analyte,
b) mettre en présence du ou des spots dudit compartiment au moins un ligand de détection d'un analyte, ledit ligand de détection d'un analyte étant couplé à un marqueur de détection direct ou indirect,
c) lorsqu'au moins un ligand de détection d'un analyte est couplé à un marqueur de détection indirect, mettre en présence du ou des spots dudit compartiment un rapporteur dudit marqueur de détection indirect,
d) lorsque le rapporteur utilisé à l'étape c) est couplé à un marqueur de détection indirect, mettre en présence du ou des spots dudit compartiment un rapporteur du marqueur de détection indirect couplé audit rapporteur utilisé à l'étape c),
e) détecter un signal produit par au moins un marqueur de contrôle résistant dans ledit compartiment,
f) définir une grille de lecture à partir de la localisation du signal détecté à l'étape e),
g) éventuellement, détecter un signal produit par au moins un marqueur de détection d'un ligand de détection d'un analyte, et
h) éventuellement, lire le signal détecté à l'étape g) sur la grille de lecture définie à l'étape f),
ledit ou lesdits marqueurs de contrôle résistants étant des marqueurs qui restent au moins partiellement fixés au niveau du spot à la surface du support solide au cours de la mise en œuvre dudit procédé de détection d'au moins un analyte, de sorte à produire un signal détectable lors de l'étape e).

Les étapes a) à d) sont toujours réalisées avant les étapes e) à h).

L'étape g) peut être réalisée avant ou après ou simultanément à l'étape e). Dans un mode de réalisation particulier, l'étape g) est réalisée avant ou après l'étape e).

L'étape f) est toujours réalisée après l'étape e).

L'étape f) est toujours réalisée après l'étape e) et avant l'étape h), et peut être réalisée avant, après ou simultanément à l'étape g).

L'étape h) est toujours réalisée ultérieurement aux étapes e) à g).

Les étapes a) à f) et, le cas échéant, l'étape g) et/ou l'étape h) sont notamment effectuées pour chaque compartiment d'un support solide comprenant au moins un spot comprenant au moins un marqueur de contrôle résistant et au moins un ligand de capture d'un analyte, dans lequel un échantillon est analysé.

Les étapes g) et/ou h) peuvent ne pas être effectuées si la grille de lecture définie à l'étape f) n'indique aucune zone pour la lecture du signal à détecter à l'étape g).

Si l'étape h) est effectuée, l'étape g) est également effectuée.

Le procédé peut avantageusement comprendre une ou plusieurs étapes de lavage, par exemple entre chaque ou certaines des étapes a) à d).

Le lavage de chaque compartiment destiné à l'analyse d'un échantillon comprend au moins un cycle, de préférence 3 à 6 cycles de distribution et d'aspiration d'un volume (par exemple 400 µL) d'une solution de lavage (par exemple une solution de tampon Tris NaCI 0,01M, pH 7,4 additionné de Tween 20 à 0,1%).

Selon un mode de réalisation particulier de l'invention, aucune opération et en particulier aucune étape de pipetage, distribution, agitation, aspiration ou lavage n'est réalisée entre les étapes e) et g), quel que soit l'ordre dans lequel les étapes e), f) et g) sont effectuées.

L'expression « mettre un composé X en présence des spots d'un compartiment » signifie que le composé X est ajouté dans un compartiment comprenant lesdits spots, ledit compartiment étant destiné à analyser un échantillon.

Lorsqu'au moins deux composés sont à mettre en présence du ou des spots d'un compartiment au cours d'une même étape et/ou lorsqu'au moins deux des étapes a) à d) sont réalisées simultanément, lesdits composés peuvent être mis en présence dudit ou desdits spots séparément, c'est-à-dire apportés sous la forme de compositions distinctes ; alternativement, lesdits composés ou certains desdits composés peuvent être mis en présence du ou des spots d'un compartiment sous la forme d'un ou plusieurs mélanges.

Le procédé de détection est notamment mis en œuvre au moyen d'un support solide tel que défini ci-dessus ou obtenu par le procédé de préparation tel que défini ci-dessus.

Le ou les marqueurs de contrôle résistant, le ou les spots, le ou les ligands de capture d'un analyte sont notamment tel que défini ci-dessus.

En particulier, le ou les marqueurs de contrôle résistants sont des marqueurs qui restent au moins partiellement fixés au niveau dudit spot à la surface du support solide au cours de la mise en œuvre dudit procédé de détection d'au moins un analyte, de sorte à produire un signal détectable lors de l'étape e), c'est-à-dire lorsque le procédé d'analyse s'est déroulé sans détérioration d'un spot.

La présente invention concerne particulièrement un procédé tel que défini ci-dessus, caractérisé en ce que ledit, lesdits ou un desdits marqueurs de contrôle résistants est (sont) un (des) fluorophore(s), par exemple une ou des molécules chimiques fluorescentes ou une ou des protéines fluorescentes, par exemple un mélange de fluorophores.

La présente invention a particulièrement pour objet un procédé tel que défini ci-dessus, caractérisé en ce que ledit, lesdits ou un desdits marqueurs de contrôle résistants est un marqueur de contrôle résistant, en particulier un fluorophore, dont le spectre d'excitation ne recouvre pas le spectre d'émission du signal émis par ou correspondant au marqueur de détection dudit ou desdits ligands de détection d'un analyte et dont le spectre d'émission ne recouvre pas ou recouvre partiellement le spectre d'émission du ou correspondant au marqueur de détection dudit ou desdits ligands de détection d'un analyte.

La présente invention a par exemple pour objet un procédé tel que défini ci-dessus, caractérisé en ce que ledit, lesdits ou un desdits marqueurs de contrôle résistants est un marqueur de contrôle résistant, en particulier un fluorophore, dont le spectre d'excitation ne recouvre pas le spectre d'émission du signal émis par un composé luminescent obtenu par réaction du luminol et/ou d'un analogue et/ou d'un dérivé du luminol ou d'un analogue du luminol avec une enzyme peroxydase et dont le spectre d'émission ne recouvre pas ou recouvre partiellement le spectre d'émission d'un composé luminescent obtenu par réaction du luminol et/ou d'un analogue et/ou d'un dérivé du luminol ou d'un analogue du luminol avec une enzyme peroxydase.

Dans un mode de réalisation préféré, le procédé de détection tel que défini ci-dessus comprend les étapes suivantes :
a) mettre un échantillon à analyser en présence du ou des spots d'un compartiment d'un support solide, ledit spot ou au moins un desdits spots comprenant au moins un marqueur de contrôle résistant et au moins un ligand de capture d'un analyte,
b) mettre en présence du ou des spots dudit compartiment au moins un ligand de détection d'un analyte, ledit ligand de détection d'un analyte étant couplé à un marqueur de détection indirect,
c) mettre en présence du ou des spots dudit compartiment un rapporteur dudit marqueur de détection indirect,
d) lorsque le rapporteur utilisé à l'étape c) est couplé à un marqueur de détection indirect, mettre en présence du ou des spots dudit compartiment un rapporteur du marqueur de détection indirect couplé audit rapporteur utilisé à l'étape c),
e) détecter un signal produit par au moins un marqueur de contrôle résistant dans ledit compartiment,
f) définir une grille de lecture à partir de la localisation du signal détecté à l'étape e),
g) éventuellement, détecter un signal produit par au moins un marqueur de détection d'un ligand de détection d'un analyte, et
h) éventuellement, lire le signal détecté à l'étape g) sur la grille de lecture définie à l'étape f).

La présente invention a particulièrement pour objet un procédé tel que défini ci-dessus, caractérisé en ce que :
- le signal produit par au moins un marqueur de détection d'un ligand de détection d'un analyte est la lumière émise par un composé chimiluminescent obtenu par réaction d'une peroxydase avec le luminol, un analogue du luminol et/ou un dérivé du luminol ou d'un analogue du luminol, et
- le signal produit par le ou lesdits marqueurs de contrôle résistants est une lumière émise en dehors des longueurs d'ondes allant de 375 nm à 550 nm, de 375 à 580 nm, ou de 350 à 580 nm (les bornes étant comprises).

De préférence, le ou les marqueurs de contrôle résistants ont un spectre d'excitation et, de préférence, un spectre d'émission en dehors des longueurs d'onde allant de 375 nm à 550 nm, de 375 à 580 nm, ou de 350 à 580 nm (bornes comprises).

Le procédé d'analyse est, par exemple, un immunotest et en particulier un test immuno-enzymatique, tel qu'un test ELISA (*Enzyme-Linked ImmunoSorbent Assay*). Le procédé d'analyse peut également être utilisé pour la détection d'acides nucléiques.

Dans l'étape a), un échantillon à analyser est mis en présence du ou des spots d'un compartiment du support solide.

Lorsque plusieurs échantillons sont analysés et que le support solide comprend plusieurs compartiments, l'étape a) comprend la mise en présence d'un échantillon avec le ou les spots d'au moins un compartiment d'un support solide, ledit support solide comprenant au moins autant de compartiments comprenant au moins un spot comprenant au moins un marqueur de contrôle résistant et au moins un ligand de capture d'un analyte que le nombre d'échantillons à analyser ou alors plusieurs supports solides sont utilisés.

Lorsque plusieurs échantillons sont analysés et que le support solide comprend un compartiment unique, l'étape a) comprend l'ajout d'un échantillon dans le compartiment d'un support solide (ou, lorsque ledit compartiment s'assimile au support solide lui-même, l'ajout d'un échantillon sur ledit support solide), et on utilise au moins autant de supports solides comprenant un compartiment comprenant au moins un spot comprenant au moins un marqueur de contrôle résistant et au moins un ligand de capture d'un analyte que le nombre d'échantillons à analyser.

Lorsque plusieurs échantillons sont analysés, un compartiment différent est utilisé pour chaque échantillon. Il est possible également d'utiliser plusieurs compartiments d'un ou plusieurs support solides pour analyser un même échantillon, en particulier si lesdits compartiments ont une composition en spots différente.

Les étapes a) et b) peuvent être réalisées simultanément, l'étape a) avant l'étape b) ou encore l'étape b) avant l'étape a). Lorsque l'étape b) est réalisée avant l'étape a), le procédé ne comprend pas d'étape de lavage entre ces deux étapes a) et b).

Lorsqu'elles sont présentes, les étapes c) et d) sont, de préférence, réalisées après les étapes a) et b) et il y a de préférence au moins une étape de lavage entre les étapes a) et b) et les étapes c) et d).

Lorsqu'elles sont présentes, les étapes c) et d) peuvent être réalisées simultanément ou l'étape c) avant l'étape d). Lorsque l'étape d) est réalisée avant l'étape c), le procédé ne comprend pas d'étape de lavage entre ces deux étapes d) et c).

Lorsqu'elle est présente, l'étape d) est de préférence réalisée après l'étape c) et il y a au moins une étape de lavage entre les étapes c) et d).

Dans l'étape b), au moins un ligand de détection d'un analyte est mis en présence du ou des spots dudit compartiment.

L'étape b) est notamment effectuée dans chaque compartiment destiné à l'analyse d'un échantillon.

Le ou les ligands de détection d'un analyte sont notamment tels que définis ci-dessus.

De préférence, l'étape b) comprend l'ajout d'au moins un ligand de détection de chaque analyte à détecter. Lorsque différents ligands de détection d'un analyte sont utilisés, ils peuvent être ajoutés simultanément ou successivement, une partie ou la totalité des ligands pouvant être apportés sous la forme de compositions distinctes ou d'un ou plusieurs mélanges. Une partie des ligands de détection d'un analyte peut être ajoutée simultanément à l'étape a), suivie de préférence par une étape de lavage avant d'ajouter le reste des ligands de détection d'un analyte.

Lorsque des ligands de détection d'un analyte sont ajoutés successivement au cours de l'étape b), chaque ajout d'au moins un ligand de détection d'analyte peut être suivi d'une étape de lavage du compartiment destiné à l'analyse d'un échantillon.

La réalisation des étapes c) et d) dépend du marqueur de détection du ligand de détection et, le cas échéant, du marqueur de détection du rapporteur du marqueur de détection du ligand de détection.

Ainsi, l'étape c) est réalisée lorsqu'au moins un ligand de détection d'un analyte est couplé à un marqueur de détection indirect.

De préférence, les ligands de détection d'un analyte sont couplés au même marqueur de détection. Si au moins deux ligands de détection d'un analyte sont couplés à des marqueurs de détection indirects différents, l'étape c) est effectuée pour chaque marqueur de détection indirect, afin de détecter le signal de chaque marqueur.

L'étape d) est réalisée lorsqu'au moins un rapporteur (également appelé premier rapporteur) utilisé à l'étape c) est couplé à un marqueur de détection indirect. Le rapporteur utilisé à l'étape d) est appelé deuxième rapporteur.

Lorsque l'étape d) est réalisée, l'étape c) est donc également réalisée

Dans l'étape e), un signal produit par au moins un marqueur de contrôle résistant est détecté dans ledit compartiment.

Si au moins deux marqueurs de contrôle résistants sont utilisés, l'étape e) comprend, de préférence, la détection d'un signal produit par chacun desdits marqueurs de contrôle résistant.

La détection du signal produit par au moins un marqueur de contrôle résistant permet notamment de localiser chaque spot comprenant au moins un marqueur de contrôle résistant dans chaque compartiment du support solide.

Le signal produit par au moins un marqueur de contrôle résistant est, de préférence, un signal émis en fluorescence.

Dans l'étape f), une grille de lecture est définie à partir de la localisation du signal détecté à l'étape e).

La grille de lecture indique précisément dans quelle(s) zone(s) du compartiment le deuxième signal doit être lu (ou « analysé » ou « pris en compte » ou « interprété). En particulier, un deuxième signal détecté à l'étape g) en dehors de la ou d'une des zones définies dans la grille de lecture n'est pas pris en compte dans la lecture (ou « l'analyse » ou « l'interprétation ») du deuxième signal lors de l'étape h).

Il est particulièrement avantageux de délimiter lesdites zones précisément d'après le contour de chaque spot.

L'étape f) de définition de la grille de lecture comprend donc notamment la vérification de la qualité des spots, à partir du signal détecté à l'étape e) qui correspond à au moins un marqueur de contrôle résistant.

Par « qualité des spots », on entend la présence, la localisation et/ou l'intégrité du ou des spots.

L'intégrité des spots comprend la taille du spot et sa forme.

Un contrôle de la qualité d'un spot est notamment positif si le spot est présent dans le compartiment à une position conforme à celle attendue, s'il a un contour bien délimité, si sa forme est conforme aux critères d'acceptabilité, par exemple, s'il s'agit d'une forme discoïdale, approximativement discoïdale, par exemple ovale ou avec une circularité supérieure à 80%, et s'il ne recoupe pas un autre spot du compartiment.

Une « circularité supérieure à 80% » signifie que le spot détecté a une circularité suffisante pour garantir qu'il n'a pas été endommagé par l'ensemble des traitements réalisés au cours du procédé d'analyse.

Par exemple, un contrôle de la qualité d'un spot est positif si un signal est détecté à l'étape e), que ce signal a un contour bien délimité, une forme discoïdale, approximativement discoïdale, par exemple ovale ou avec une circularité supérieure à 80%, et s'il ne recoupe pas le signal produit par un autre spot du compartiment.

Si aucun signal n'est détecté à l'étape e) au niveau d'un spot ou si le spot ne répond pas au contrôle de qualité pour au moins un autre des motifs indiqués ci-dessus, le deuxième signal éventuellement détecté à l'étape g) au niveau dudit spot n'est pas pris en considération lors de la lecture du deuxième signal à l'étape h), de même éventuellement que le deuxième signal détecté au niveau de chaque spot du compartiment correspondant audit spot.

Dans les étapes e) et g), la détection d'un signal comprend, de préférence, l'acquisition d'un signal.

L'utilisation d'un ou plusieurs marqueur(s) de contrôle résistant(s) selon l'invention permet ainsi d'effectuer une lecture du deuxième signal aux emplacements précis où sont localisés les spots, permettant d'améliorer la sensibilité de l'analyse, tout en sécurisant les résultats rendus, en particulier en permettant d'écarter des résultats faussement positifs ou faussement négatifs liés à un défaut de spot.

La présente invention a particulièrement pour objet le procédé tel que défini ci-dessus, caractérisé en ce que le signal produit par au moins un marqueur de détection d'un ligand de détection d'un analyte est un signal luminescent, en particulier un signal chimiluminescent, par exemple la lumière émise par réaction du luminol et/ou d'un analogue et/ou d'un dérivé du luminol ou d'un analogue du luminol avec une enzyme peroxydase.

La présente invention a plus particulièrement pour objet un procédé tel que défini ci-dessus, caractérisé en ce que le signal produit par au moins un marqueur de contrôle résistant est détecté en fluorescence et le signal produit par au moins un marqueur de détection d'un ligand de détection d'un analyte est détecté en luminescence, en particulier en chimiluminescence.

Dans un mode de réalisation préféré, au moins les étapes e) à h) sont mises en œuvre au moyen d'un même dispositif, par exemple un dispositif tel que décrit dans la section « dispositif pour une détection améliorée ».

Dans un autre mode de réalisation préféré, l'ensemble des étapes du procédé est mis en œuvre au moyen d'un même dispositif, par exemple un dispositif tel que décrit dans la section « dispositif pour une détection améliorée ».

### Procédé de sélection d'un marqueur de contrôle résistant

La présente invention a également pour objet un procédé de sélection d'un marqueur de contrôle résistant comprenant les étapes suivantes :
a) déposer un marqueur à tester à la surface d'un support solide, pour former au moins un spot,
b) éventuellement saturer la surface du support solide,
c) éventuellement sécher la surface du support solide,
d) réaliser au moins une des étapes suivantes :
   (i) réaliser une ou plusieurs étapes de lavage,
   (ii) mettre le ou les spots en présence d'un ligand de détection,
   (iii) mettre le ou les spots en présence d'un rapporteur,
   (iv) mettre le ou les spots en présence d'un substrat,
e) sélectionner un marqueur qui produit un signal à l'issue de l'étape d).

Le marqueur à tester est un composé qui produit (par exemple émet) un signal pouvant être détecté. Le marqueur à tester est un fluorophore.

Le support solide est notamment tel que défini ci-dessus.

L'étape a) comprend ou consiste à déposer un marqueur à tester à la surface d'un support solide, pour former au moins un spot.

Le dépôt du marqueur peut être réalisé manuellement ou de manière automatisée au moyen d'un dispositif approprié.

Le marqueur à tester est généralement déposé sous la forme d'une solution comprenant ledit marqueur.

L'étape a) peut comprendre le dépôt d'un même marqueur à tester dans plusieurs spots, à des concentrations croissantes.

La solution déposée à l'étape a) peut comprendre ou non un ligand de capture.

Le procédé comprend une éventuelle étape b) de saturation de la surface du support solide. L'étape de saturation a notamment pour objet d'empêcher la fixation non spécifique de composés, lors de la mise en œuvre du procédé d'analyse.

Dans l'étape c), la surface du support solide est éventuellement séchée.

Généralement, l'étape a) et/ou l'étape b) sont suivies d'une ou plusieurs étapes a') et/ou b') de lavage.

L'étape d) comprend ou consiste à réaliser au moins une des étapes suivantes, de préférence au moins deux des étapes suivantes, plus préférentiellement au moins trois des étapes suivantes :
(i) réaliser une ou plusieurs étapes de lavage,
(ii) mettre le ou les spots en présence d'au moins un ligand de détection,
(iii) mettre le ou les spots en présence d'au moins un rapporteur, et/ou
(iv) mettre le ou les spots en présence d'au moins un substrat.

Dans un mode de réalisation préféré, l'étape d) comprend ou consiste en l'étape (iii) et/ou l'étape (iv), de préférence l'étape (iii) et l'étape (iv).

Dans un autre mode de réalisation préféré, l'étape d) comprend ou consiste en l'étape (i), l'étape (iii) et/ou l'étape (iv), de préférence l'étape (i), l'étape (iii) et l'étape (iv).

Dans un mode de réalisation préféré, l'étape d) comporte au moins l'étape (i), l'étape (ii), l'étape (iii) et l'étape (iv).

Les étapes (i) à (iv) peuvent être réalisées dans n'importe quel ordre souhaité.

Toutefois, dans un mode de réalisation préféré, l'étape d) comprend les étapes suivantes et dans l'ordre suivant : étape (ii), étape (i), étape (iii), étape (i), étape (iv) et étape (i).

Dans un autre mode de réalisation préféré, l'étape d) comprend les étapes suivantes et dans l'ordre suivant : étape (ii), étape (i), étape (iii), étape (i) et étape (iv).

Dans un autre mode de réalisation préféré, lorsque ces étapes sont présentes, l'étape (ii) est réalisée avant l'étape (iii) et/ou avant l'étape (iv) et l'étape (iii) est réalisée avant l'étape (iv). L'étape (i) peut être réalisée entre les étapes (ii) et (iii), (ii) et (iv) (en particulier lorsque l'étape (iii) est absente) et/ou (iii) et (iv).

De préférence, le ou un des rapporteurs de l'étape (iii) est le rapporteur d'un marqueur de détection couplé à au moins un ligand de détection de l'étape (ii) et/ou le ou un des substrats de l'étape (iv) est un rapporteur d'un marqueur de détection couplé à un rapporteur.

Par exemple, le ou un des ligands de détection de l'étape (ii) est couplé à un marqueur de détection du type biotine, le ou un des rapporteur de l'étape (iii) est un rapporteur du type streptavidine couplé à une enzyme, par exemple une peroxydase, et/ou le ou un des substrats de l'étape (iv) est un substrat de ladite enzyme, par exemple le luminol, un analogue du luminol, et/ou un dérivé du luminol ou d'un analogue du luminol.

Le procédé de sélection comprend une étape e) de sélection d'un marqueur qui produit un signal à l'issue de l'étape d). En particulier, le marqueur sélectionné à l'étape e) produit un signal détectable à l'issue de l'étape d). Un « signal détectable » est notamment tel que défini ci-dessus.

De préférence, l'étape e) comprend en outre la sélection d'un marqueur qui n'interfère pas ou peu avec la détection du signal produit par un marqueur de détection d'un ligand de détection.

Le procédé de sélection selon l'invention peut également comprendre :
- éventuellement dans un premier temps :
   ∘ les étapes a) à c) ci-dessus, dans lesquelles le ou les spots formés à l'étape a) ne comprennent pas de ligand de capture,
   ∘ éventuellement une première étape de présélection comprenant la sélection d'un marqueur qui produit un signal détectable à l'issue de l'étape c),
   ∘ éventuellement les étapes d) à e) sur le même support, de préférence lesdites étapes n'étant réalisées qu'avec ledit marqueur sélectionné à la première étape de présélection, suivies d'une deuxième étape de présélection comprenant la sélection d'un marqueur qui produit un signal détectable à l'issue de l'étape d),
- dans un deuxième temps, les étapes a) à e) ci-dessus, dans lesquelles le ou les spots formés à l'étape a) comprennent un ligand de capture, les étapes d) à e) étant, de préférence, réalisées uniquement avec un marqueur sélectionné à ladite première étape de présélection et/ou sélectionné à ladite deuxième étape de présélection.

### Utilisation d'un marqueur contrôle résistant

La présente invention a également pour objet l'utilisation d'au moins un marqueur de contrôle résistant dans au moins un spot destiné à détecter un analyte, pour sécuriser un procédé de détection d'au moins un analyte dans un échantillon ou au moins un échantillon.

Par « sécuriser un procédé de détection d'au moins un analyte dans un échantillon ou au moins un échantillon », on entend ici garantir la fiabilité des résultats obtenus à l'issue dudit procédé de détection, en particulier en évitant la présence de « faux négatifs » ou de « faux positifs ».

Un « faux négatif » est un résultat négatif traduisant l'absence d'un ou de plusieurs analytes à détecter dans un échantillon, alors que ledit ou lesdits analytes étaient présents dans l'échantillon et auraient dû être détectés.

Un « faux positif » est un résultat positif traduisant la présence d'un ou de plusieurs analytes à détecter dans un échantillon, alors que ledit ou lesdits analytes étaient absents dans l'échantillon.

La sécurisation du procédé de détection d'au moins un analyte dans un échantillon est notamment obtenue en contrôlant, à l'issue du procédé de détection, la qualité du ou des spots destinés à la détection d'un analyte et/ou en améliorant la sensibilité de la détection des analytes, grâce à l'utilisation d'au moins un marqueur de contrôle résistant.

La présente invention a ainsi pour objet l'utilisation d'au moins un marqueur de contrôle résistant dans au moins un spot destiné à détecter un analyte pour sécuriser un procédé de détection d'au moins un analyte dans un échantillon ou au moins un échantillon, caractérisée en ce qu'elle comprend :
- le contrôle de la qualité dudit spot, après que ledit spot ait été mis en présence de l'échantillon et d'au moins un ligand de détection d'un analyte à détecter, en particulier à la fin du procédé d'analyse, et/ou
- la lecture du signal produit par au moins un marqueur de détection d'un ligand de détection d'un analyte à détecter sur une grille de lecture définie à partir de la localisation du signal produit par ledit ou lesdits marqueurs de contrôle résistants.

La lecture du signal produit par au moins un marqueur de détection d'un ligand de détection d'un analyte à détecter est donc réalisée sur une grille de lecture définie à partir de la localisation du signal produit par ledit ou lesdits marqueurs de contrôle résistants et détecté à la fin du procédé d'analyse.

La présente invention a particulièrement pour objet l'utilisation d'au moins un marqueur de contrôle résistant dans au moins un spot destiné à détecter un analyte pour sécuriser un procédé de détection d'au moins un analyte dans un échantillon ou au moins un échantillon, caractérisée en ce qu'elle comprend :
- le contrôle de la qualité dudit spot, après que ledit spot ait été mis en présence de l'échantillon, d'au moins un ligand de détection d'un analyte à détecter couplé à un marqueur de détection indirect, un rapporteur (appelé également premier rapporteur) dudit marqueur de détection indirect, éventuellement un deuxième rapporteur d'un marqueur de détection indirect couplé audit premier rapporteur, et/ou
- la lecture du signal produit par au moins un marqueur de détection d'un ligand de détection d'un analyte à détecter sur une grille de lecture définie à partir de la localisation du signal produit par ledit ou lesdits marqueurs de contrôle résistants.

La présente invention a particulièrement pour objet l'utilisation telle que définie ci-dessus, caractérisée en ce que le contrôle de la qualité du spot comprend ou consiste à contrôler la présence, la localisation et/ou l'intégrité du spot.

La grille de lecture indique dans quelle(s) zone(s) du compartiment le signal produit par le ou les marqueurs de détection d'un ligand de détection d'un analyte doit être lu.

D'autres caractéristiques et avantages de l'invention ressortiront mieux au travers des exemples qui suivent, donnés à titre illustratif et non limitatif.

### Figures

Figure 1 : Utilisation de différents fluorophores et images résultantes en fluorescence à différentes étapes du protocole décrit dans l'exemple 1.
Figure 2 : Acquisition du signal de fluorescence de 12 puits comprenant 9 spots à l'issue d'un procédé d'analyse selon l'invention.
Figure 3 : Image en fluorescence : Comparaison de la grille de lecture théorique (cercles blancs continus) par rapport à la position réelle des spots en fluorescence (cercles blancs en pointillés) sur la base du signal produit par le marqueur de contrôle résistant.
Figure 4 : Image en chimiluminescence : Amélioration de la justesse du procédé d'analyse en définissant la grille de lecture sur la position réelle des spots détectée grâce au marqueur de contrôle résistant (traits en pointillés), versus la grille de position théorique (traits continus).
Figure 5 : Image en chimiluminescence : Amélioration de la justesse du procédé d'analyse en vérifiant l'intégrité des spots détectés avant de valider le rendu de résultat.

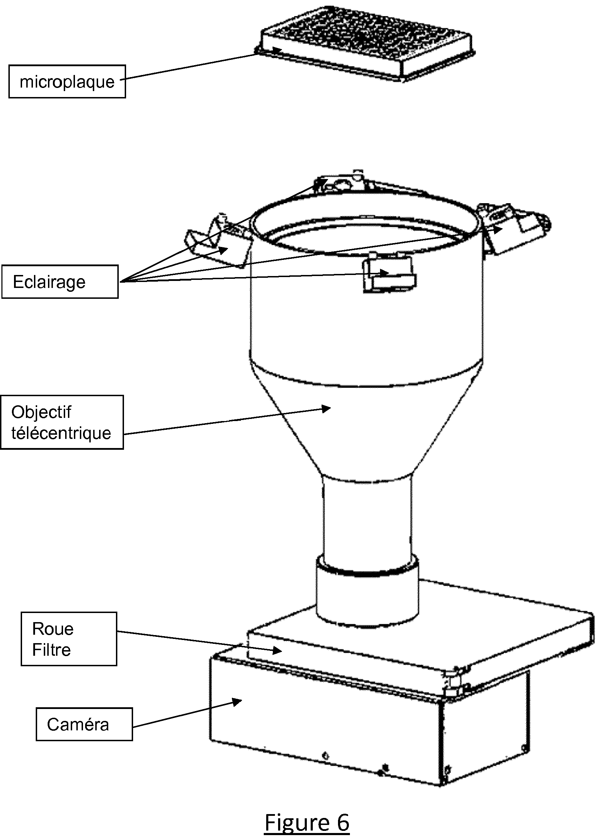

Figure 6 : Schéma d'un banc optique comprenant un système d'éclairage, un objectif télécentrique, une roue filtre, ledit objectif télécentrique étant couplé sur sa lentille de sortie à la roue filtre et une caméra.

### Exemples

### Exemple 1 : Exemple de procédé de sélection d'un marqueur de contrôle résistant

### Matériel et Méthode

Au sein de chaque puits d'une microplaque en polystyrène (Greiner, Allemagne) sont déposées, puits à puits, des gouttes de 500 nl d'une solution de fluorophore dans le tampon utilisé classiquement pour effectuer le spotting des antigènes ou anticorps. Les fluorophores suivants sont utilisés dans cet exemple : Atto 633-acide carboxylique (i.e.Atto 633-COOH ou Atto 633) (fournisseur : ATTO-TEC ; Allemagne), Atto 633-amine (i.e. dérivé amine de l'Atto 633) (fournisseur : ATTO-TEC ; Allemagne), Dye 634-acide carboxylique (i.e. Dye 634-COOH ou Dye 634) (fournisseur : Dyomics, Allemagne), Dye 634-amine (i.e. dérivé amine du Dye 634) (fournisseur : Dyomics, Allemagne), Dye 630-amine (i.e. dérivé amine du Dye 630) (fournisseur : Dyomics, Allemagne), APC (Allophycocyanine) (fournisseur : Febico ; Taïwan), B-Phycoerythrine (Febico ; Taïwan). Le fond de chaque puits de ces microplaques possède des capacités d'adsorption de molécules en soi connu de l'homme du métier. La surface de chaque puits ainsi obtenus est saturée avec une solution de saturation en soi connu de l'homme du métier ; les puits sont remplis par la solution de saturation ; la solution de saturation est retirée et les plaques sont ensuite séchées ; après une étape de réhydratation de ces plaques, une solution de substrat contenant le lumino (ELISTAR ETA C Ultra ELISA (Cyanagen, Italie) *(cf. exemple 2)* est alors ajoutée à raison de 50µl/puits. Les images en fluorescence sont réalisées aux différentes étapes du protocole décrit ci-dessus (c'est-à-dire après les étapes de dépôt des gouttes, de saturation, de séchage, de réhydratation, et après l'ajout de la solution de substrat contenant le luminol), en utilisant le système Chemidoc™ MP System (Bio-Rad) possédant les filtres appropriés aux fluorophores.

### Résultats

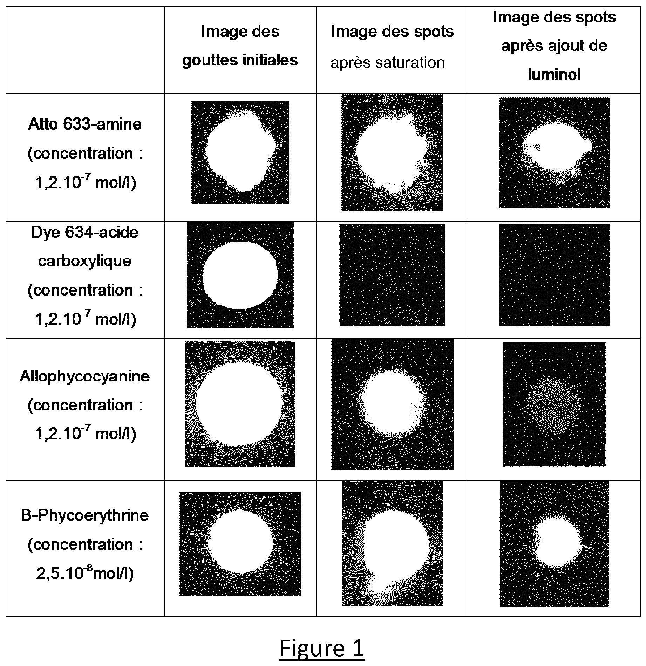

La figure 1 décrit l'utilisation de différents fluorophores et les images résultantes à différentes étapes du protocole décrit ci-dessus.

La fluorescence obtenue avec les 4 fluorophores est parfaitement visible à l'issue du dépôt des gouttes sur la plaque. Cette fluorescence persiste pour l'Atto 633-amine, pour l'allophycocyanine et pour la B-Phycoerythrine après élimination de la solution de saturation (cf. image des spots après saturation). Elle persiste également après ajout de la solution de substrat contenant le luminol pour l'Atto 633-amine et pour la B-Phycoerythrine, de façon très faible pour l'allophycocyanine. Des résultats similaires sont obtenus avec le Dye 634-amine et le Dye 630-amine (résultats non montrés). En revanche, aucune fluorescence résiduelle n'est visible à l'issue de l'étape de lavage avec le Dye 634-acide carboxylique et a fortiori après ajout de la solution de substrat, de même que pour l'Atto 633-acide carboxylique (résultats non montrés).

Ainsi, l'Atto 633-amine, le Dye 634-amine, le Dye 630-amine et la B-Phycoerythrine sont des marqueurs de contrôle résistants selon la présente invention.

Par ailleurs, comme illustré ci-après dans l'exemple 2, le Dye 634 couplé à la-BSA est également un marqueur de contrôle résistant selon l'invention.

### Exemple 2 : Absence d'impact de la présence d'un fluorophore de contrôle résistant sur la détection d'un analyte d'intérêt

### Matériel et Méthode

### (i) Préparation de la microplaque

Au sein de chaque puits d'une microplaque en polystyrène (Greiner, Allemagne) sont déposés par rangées à l'aide d'un robot spotteur, des gouttes de 50 nL d'une solution contenant le/les ligands de capture ainsi qu'un fluorophore sélectionné selon le procédé décrit dans la demande.

La solution de ligand de capture peut être :
- soit un antigène ou mélange d'antigènes pouvant être constitué d'une protéine recombinante associée à un ou des peptides synthétiques dans le cadre d'un test de détection d'anticorps,
- soit un anticorps ou un mélange d'anticorps dirigés contre le marqueur recherché dans le cas d'un test de détection d'antigène.

Ces solutions de ligand de capture contiennent le fluorophore sélectionné, à titre d'exemple l'Atto 633-amine (Atto-tec, Allemagne) ou un complexe Dye 634-BSA, obtenu selon un protocole connu de l'homme du métier à partir de Dye 634 (Dyomics, Allemagne) sous forme d'ester-NHS (ester-N-Hydroxysuccinimide) couplé à la BSA ; ces fluorophores sont ajoutés à la dose appropriée, déterminée pour chacun ; pour le Dye 634-BSA, la dose indiquée correspond à la concentration du complexe Dye 634-BSA. Le fond de chaque puits de ces microplaques possède des capacités d'adsorption de ces différentes protéines, en soi connu de l'homme du métier.

Les spots ainsi obtenus sont saturés avec une solution de saturation en soi connu de l'homme du métier. Les plaques sont ensuite séchées.

### (ii) Mise en œuvre du procédé d'analyse

Description des différents éléments utilisés lors de la mise en œuvre du procédé d'analyse :
Rapporteur
   Le rapporteur Streptavidine-POD (S-POD) est de la streptavidine (Roche, Allemagne) couplée à la Peroxydase (Roche Allemagne) selon la méthode décrite par Nakane et Kawaoi [J Histochem Cytochem (1974) Vol. 22, No. 12. pp. 1084-1091] en soi connu de l'homme du métier.
Solution de lavage
   Solution de tampon Tris 10 mM, pH 7,4 contenant : NaCI 218 mM, Tween 20™ (marque de la société Sigma) à 0,1%, Proclin 300™ (marque de la société Supelco) à 0,002%.
Substrat de révélation
   Le substrat de révélation ELISTAR ETA C Ultra ELISA (Cyanagen, Italie) se compose de deux solutions : XLSE024L Luminol enhancer solution (A) et XLSE024P Peroxide solution (B).

Description des différentes étapes réalisées :
Le protocole d'essai comprend les étapes suivantes :
Etape 1 :
1. Dans chaque puits d'une microplaque (comprenant les spots) sont distribués :
   - 40 µl d'échantillon : l'échantillon peut être par exemple un sérum ou un échantillon de contrôle
   - 40 µl de diluant
2. Le mélange est mis en incubation pendant 40 minutes à 37°C sous agitation.
3. Trois lavages successifs avec au moins 300 µl de solution de lavage sont réalisés.
4. S'ensuivent une étape d'incubation en présence du ligand de détection puis de lavage dans les mêmes conditions que le point 3.
5. Puis une étape d'incubation du rapporteur puis lavage.
6. Enfin une ultime étape de révélation, comportant l'ajout de 25µl de chacune des solutions de substrat de révélation B et A.
7. Le mélange est mis en incubation pendant 1 minute à 37°C sous agitation.
8. Les lectures sont réalisées avec un lecteur Chemidoc™ pour la mesure de la fluorescence et un lecteur Qview™ pour la mesure de la chimiluminescence. Les résultats des lectures sont directement traités par un système d'analyse d'image et enregistrés en Unités Relatives de Luminescence ou Relative light Unit (RLU) ; également intensité relative de fluorescence ou Relative Fluorescence Intensity (RFI).

### Résultats

Chaque donnée correspond à une moyenne d'un triplicat; les valeurs de fluorescence correspondent au niveau de signal de fluorescence déduction faite du bruit de fond.

### Résultats obtenus avec le fluorophore Atto 633-amine ajouté dans la solution d'anticorps spécifique du marqueur d'intérêt à détecter :

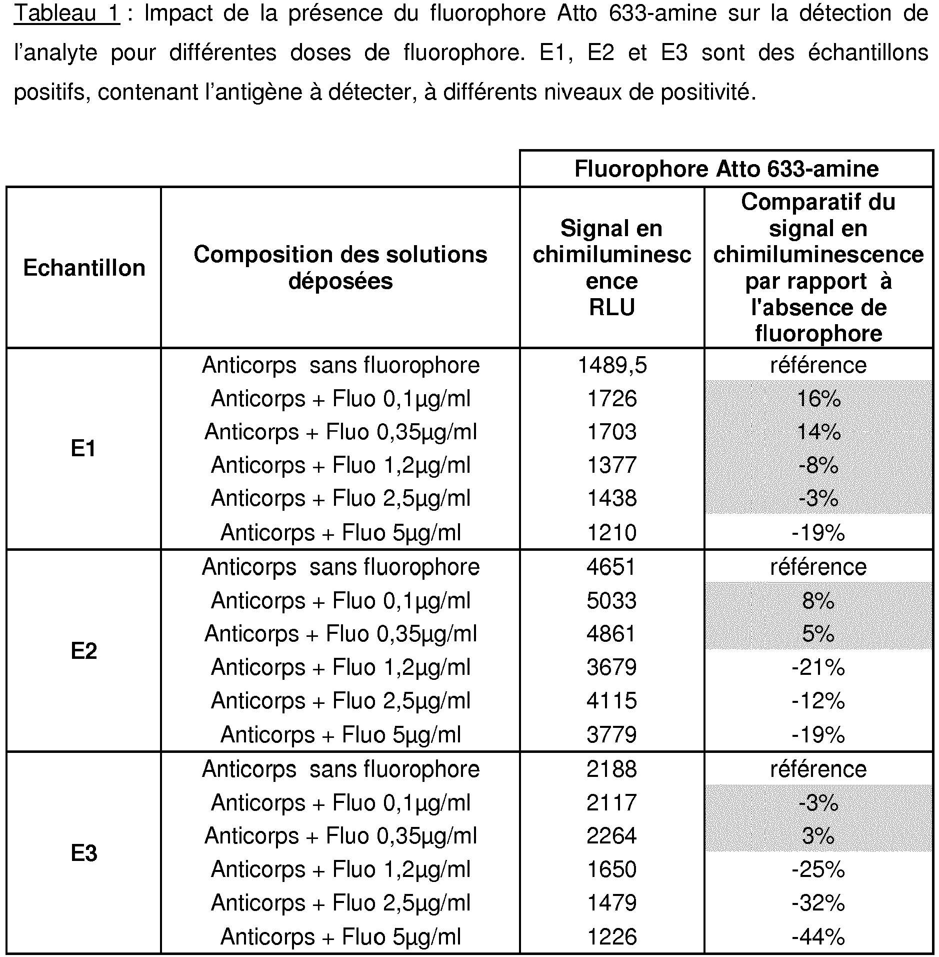

Les résultats présentés dans le tableau 1 montrent l'absence d'impact de la présence du fluorophore Atto 633-amine sur la détection de l'analyte pour des doses de fluorophore de 0,35µg/ml ou inférieures. Le calcul de la limite de détection de l'analyte montre l'absence d'impact de la présence du fluorophore jusqu'à une dose de 1,2µg/ml. La présence des ligands de capture ne modifie que très faiblement la fluorescence (cf. *tableau 2*), qui reste très importante et parfaitement détectable en fin d'analyse.

**Tableau 2 : Impact de la présence des ligands de capture sur le signal de fluorescence (cas du fluorophore Atto 633-amine). E1 et E3 sont des échantillons positifs, contenant l'antigène à détecter, à différents niveaux de positivité. N1 est un échantillon négatif, ne contenant pas l'antigène à détecter. 'Fluo' signifie Fluorophore.**

| | | **Fluorophore Atto 633-NH2** | | |
|---|---|---|---|---|
| **Echantillon** | **Composition des solutions déposées** | **Signal de fluorescence RLU** | **Signal en chimiluminescence RLU** | **Comparatif du signal de fluorescence par rapport à l'absence de marqueurs d'intérêt** |
| **E1** | Fluorophore seul 0,1µg/ml | 26843 | 54 | -21% |
| | Anticorps + Fluo 0,1µg/ml | 21158 | 1726 | |
| **E3** | Fluorophore seul 0,1µg/ml | 29039 | 56 | -31% |
| | Anticorps + Fluo 0,1 µg/ml | 20021 | 2117 | |
| **N1** | Fluorophore seul 0,1µg/ml | 24999 | 57 | -29% |
| | Anticorps + Fluo 0,1µg/ml | 17641 | 49 | |

### Résultats obtenus avec le fluorophore Dve 634-BSA ajouté dans la solution d'anticorps spécifique du marqueur d'intérêt à détecter :

Les résultats présentés dans le tableau 3 montrent l'absence d'impact de la présence du fluorophore Dye 634-BSA sur la détection de l'analyte pour des doses de fluorophore de 6µg/ml ou inférieures, dose compatible avec la détection de la fluorescence en fin d'analyse et la mise en œuvre du procédé de traitement des données tel que décrit dans la présente demande.

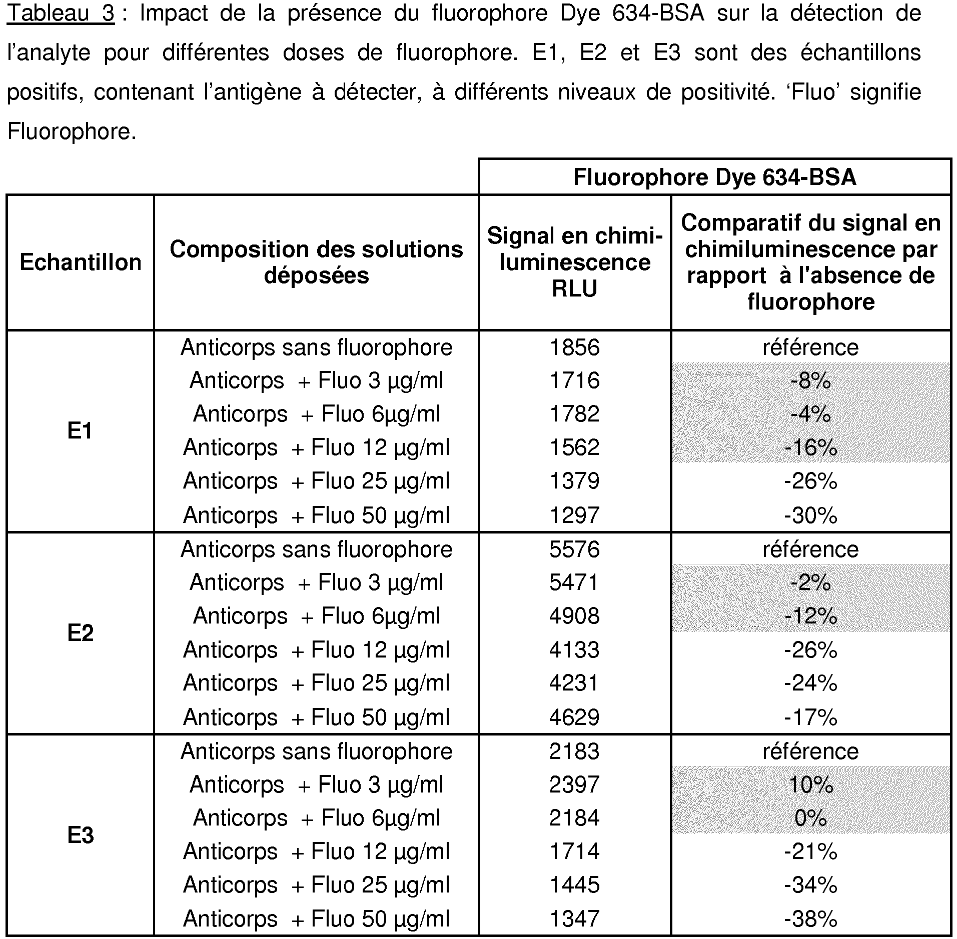

### Résultats obtenus avec le fluorophore Atto 633-amine ajouté dans la solution d'antigènes correspondant aux anticorps à détecter :

Les résultats présentés dans le tableau 4 montrent l'absence d'impact de la présence du fluorophore Atto 633-amine sur la détection de l'analyte pour la dose de fluorophore de 0,1µg/ml. La présence des ligands de capture *(cf. tableau 5)* modifie la fluorescence, mais le signal reste très important, parfaitement détectable en fin d'analyse et utilisable pour mettre en œuvre le procédé de traitement des données tel que décrit dans la présente demande.

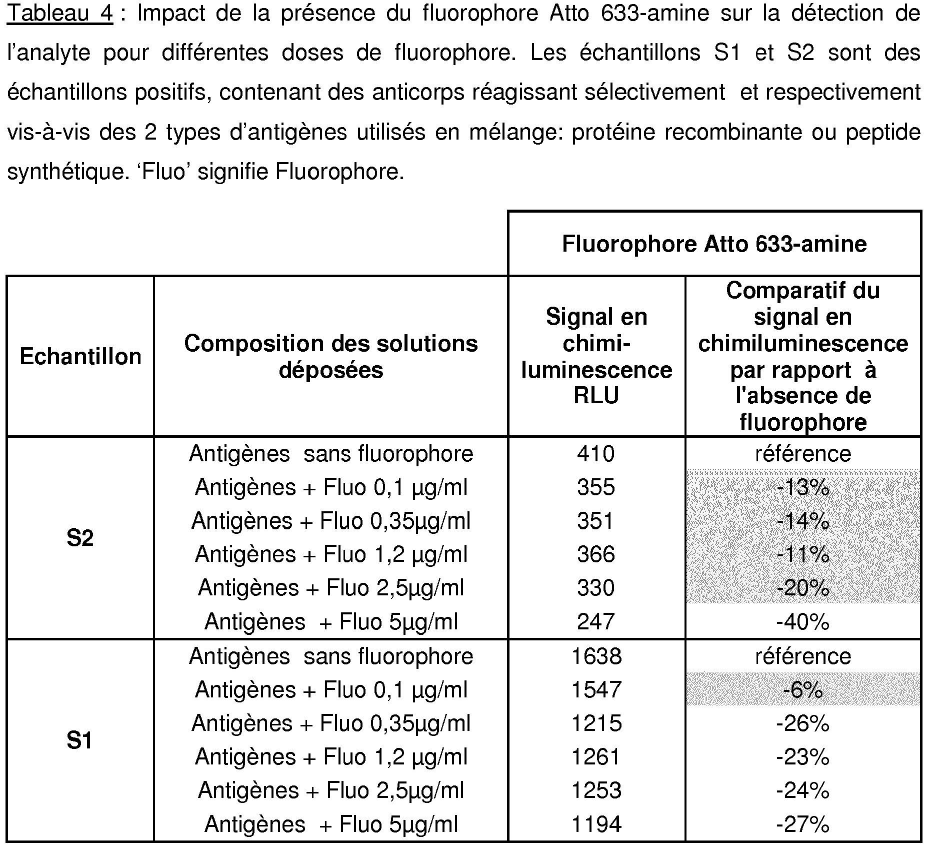

**Tableau 5 : Impact de la présence des ligands de capture sur le signal de fluorescence (cas du fluorophore Atto 633-amine). Les échantillons S1 et S2 sont des échantillons positifs, contenant des anticorps réagissant sélectivement et respectivement vis-à-vis des 2 types d'antigènes utilisés : protéine recombinante ou peptide synthétique. N2 est un échantillon négatif, ne contenant pas d'anticorps reconnaissant les antigènes utilisés. 'Fluo' signifie Fluorophore.**

| | | **Fluorophore Atto 633-amine** | | |
|---|---|---|---|---|
| **Echantillon** | **Composition des solutions déposées** | **signal de fluorescence RLU** | **Signal en chimiluminescence RLU** | **Comparatif du signal de fluorescence par rapport à l'absence de marqueurs d'intérêt** |
| **N2** | Fluorophore seul 0,1µg/ml | 70881 | 70 | -47% |
| | Antigènes + Fluo 0,1 µg/ml | 37781 | 67 | |
| **S2** | Fluorophore seul 0,1µg/ml | 72852 | 93 | -45% |
| | Antigènes + Fluo 0,1 µg/ml | 39877 | 355 | |
| **S1** | Fluorophore seul 0,1µg/ml | 73721 | 117 | -47% |
| | Antigènes + Fluo 0,1 µg/ml | 39096 | 1547 | |

### Résultats obtenus avec le fluorophore Dve 634-BSA ajouté dans la solution d'antigènes correspondant aux anticorps à détecter :

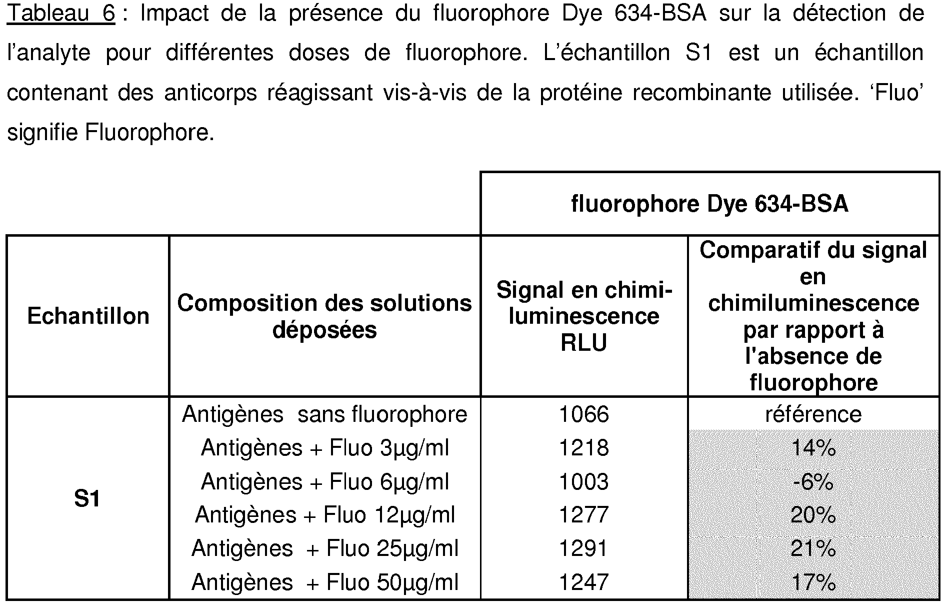

Les résultats présentés dans le tableau 6 montrent l'absence d'impact de la présence du fluorophore Dye 634-BSA sur la détection de l'analyte quelle que soit la dose de fluorophore. Le signal de fluorescence obtenu en fin d'analyse est parfaitement détectable et utilisable pour mettre en œuvre le procédé de traitement des données tel que décrit dans la présente demande.

### Exemple 3 : Utilisation d'un marqueur de contrôle résistant pour sécuriser un procédé de détection d'au moins un analyte dans un échantillon

### Matériel et Méthode

### (i) Préparation de la microplaque

Au sein de chaque puits d'une microplaque en polystyrène (Greiner, Allemagne) sont déposés par rangées à l'aide d'un robot spotteur, des gouttes de 50 nL d'une solution contenant le/les ligands de capture ainsi qu'un fluorophore sélectionné selon le procédé décrit dans la demande.
La solution de ligand de capture peut être :
- soit un antigène ou mélange d'antigènes pouvant être constitué d'une protéine recombinante associée à un ou des peptides synthétiques dans le cadre d'un test de détection d'anticorps,
- soit un anticorps ou un mélange d'anticorps dirigés contre le marqueur recherché dans le cas d'un test de détection d'antigène.

Ces solutions de ligand de capture contiennent le fluorophore sélectionné Atto 633-amine (Atto-tec, Allemagne) à la dose appropriée comprise entre 0,1 à 0,5 µg/mL. Le fond de chaque puits de ces microplaques possède des capacités d'adsorption de ces différentes protéines, en soi connu de l'homme du métier.

Les spots ainsi obtenus sont saturés avec une solution de saturation en soi connu de l'homme du métier. Les plaques sont ensuite séchées.

### (ii) Mise en œuvre du procédé d'analyse

Description des différents éléments utilisés lors de la mise en œuvre du procédé d'analyse :
I. Rapporteur
   Le rapporteur Streptavidine-POD (S-POD) est de la streptavidine (Roche, Allemagne) couplée à la Peroxydase (Roche Allemagne) par la méthode décrite par P. Nakane et A. Kawaoi [J Histochem Cytochem (1974) Vol. 22, No. 12. pp. 1084-1091] en soi connu de l'homme du métier.
II. Diluants
   a) Diluant étape 1
      Solution de tampon Tris 50 mM, pH 7,5 contenant : NaCl 150 mM, EDTA 20 mM, IgG de souris (Meridian) à 500 µg/mL, Lait de vache (100% écrémé) à 15%, Sérum de mouton à 10%, NaN3 à 0,095%.
   b) Diluant des conjugués 1
      Solution de Tampon Tris 50 mM, pH 7,5 contenant : NaCl 150 mM ; EDTA 20 mM, Chaps 0,1%, Glycérol 10%, NaN3 à 0,095%.
   c) Diluant des conjugués 2
      Solution de tampon citrate 50 mM, pH 6,7 contenant : NaCI 150 mM, EDTA 5,6 mM, Triton à 2%, Sérum de mouton à 10%, IgG de souris 500 µg/mL, Proclin 300™ (marque de la société Supelco) à 0,5%, lait de vache (100% écrémé) à 15%, Glycérol 10%. NaN3 à 0,095%.
   d) Diluant du rapporteur Streptavidine-POD
      Solution de tampon citrate 50 mM, pH 6,7 contenant : NaCl 2053 mM, Tween 20™ (marque de la société Sigma) à 0,5%, Proclin 300™ (marque de la société Supelco) à 0,5%, lait de vache (100% écrémé) à 7%, Glycérol 20%.
   e) Solution de lavage
      Solution de tampon Tris 10 mM, pH 7,4 contenant : NaCI 218 mM, Tween 20™ (marque de la société Sigma) à 0,1%, Proclin 300 ™ (marque de la société Supelco) à 0,002%.
   f) Substrat de révélation
      Le substrat de révélation ELISTAR ETA C Ultra ELISA (Cyanagen, Italie) se compose de deux solutions : XLSE024L Luminol enhancer solution (A) et XLSE024P Peroxide solution (B).
III. Cuvettes réactionnelles
   Les réactions immunologiques ont lieu dans des microplaques 96 puits (Greiner, Allemagne) en polystyrène ayant un volume maximum de 392 µL par puits.
IV. Echantillons
   Les échantillons négatifs (sérum ou plasma) utilisés proviennent de l'établissement français du sang de Lille.
V. Banc optique
   Le banc optique utilisé est constitué des éléments suivant :
   - un système d'éclairage émettant une lumière rouge centrée sur la longueur d'onde de 620nm et assemblé de tel manière qu'il illumine la face inférieure de la microplaque de manière homogène,
   - un objectif télécentrique réalisé pour permettre d'imager l'ensemble de la surface de la microplaque,
   - une roue filtre insérée entre la lentille de sortie de l'objectif télécentrique et la caméra,
   - une caméra ayant la capacité de réaliser des images avec des temps d'exposition compris entre 0,001 seconde et 250 secondes.
   - un châssis porteur qui supporte et positionne l'ensemble des éléments dont la microplaque.
   Le banc optique est étudié et assemblé de telle sorte qu'il prend les images de la face inférieure de la microplaque. La mise au point de l'objectif est faite de telle manière que la face interne des puits de microplaque est nette.
   La roue filtre est capable de présenter deux filtres différents :
   - un filtre centré sur la longueur d'onde de 680nm permettant de ne laisser passer que le signal correspondant à la lumière émise par le fluorophore,
   - un filtre permettant de laisser passer l'ensemble des longueurs d'onde compris entre 400nm et 700nm.

Description des différentes étapes réalisées :
Le protocole d'essai comprend les étapes suivantes :
Etape 1 :
   1. Dans chaque puits d'une microplaque (comprenant les spots) sont distribués successivement :
      + 20 µL de diluant étape 1
      + 20 µL de diluant des conjugués 1 comprenant les ligands de détection des analytes à doser de la première étape
      + 40 µL d'échantillon
   2. Le mélange est mis en incubation pendant 40 minutes à 37°C sous agitation.
   3. Trois lavages successifs avec au moins 400 µL de solution de lavage sont réalisés.
Etape 2 :
   4. Dans chaque puits réactionnel sont distribués 50 µL de diluant des conjugués 2 contenant les ligands de détection des analytes à doser de la deuxième étape.
   5. Le mélange est mis en incubation pendant 15 minutes à 37°C sous agitation.
   6. Les étapes de lavage (idem point 3) sont réalisées.
Etape 3 :
   7. 50 µL du rapporteur S-POD sont distribués dans chaque puits réactionnel.
   8. Le mélange est mis en incubation pendant 15 minutes à 37°C sous agitation.
Etape 4 :
   9. 25 µL de solution de révélation « B » sont distribués dans chaque puits réactionnels.
   10. 25 µL de solution de révélation « A » sont distribués dans chaque puits réactionnels.
   10. Le mélange est mis en incubation pendant 1 minute à 37°C sous agitation.
   11. L'acquisition du signal de fluorescence est réalisée pendant 10 secondes.
   12. L'acquisition du signal de luminescence est réalisée pendant 180 secondes.

### Résultats

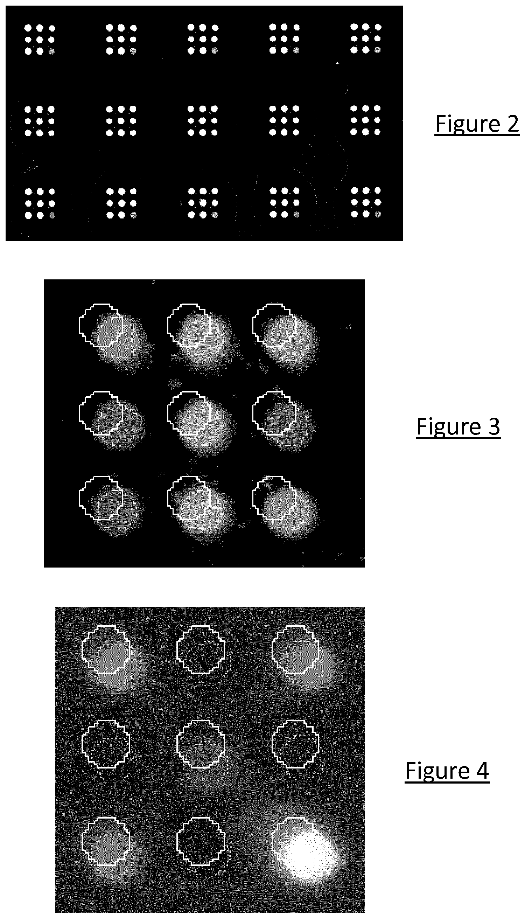

A) Persistance du marqueur de contrôle résistant après un procédé d'analyse.
Le signal fluorescent du marqueur de contrôle résistant de chacun des 9 spots présents dans les 12 puits est clairement identifiable et parfaitement mesurable à l'issu du procédé d'analyse sur la figure 2.
B) Importance de redéfinir la grille de lecture à l'issue du procédé d'analyse : comparaison des régions d'intérêt obtenues en fluorescence en définissant la grille de lecture par rapport à des positions théoriques versus en définissant la grille de lecture à partir du signal émis en fluorescence par le marqueur de contrôle résistant.
Dans la figure 3, les cercles blancs continus montrent la position théorique des spots, les cercles blancs en pointillés montrent la position réelle détectée. Les spots sont clairement décalés par rapport à leur position théorique attendue.
Cette image démontre la pertinence de la méthode qui permet de toujours cibler la position du spot réel détecté grâce au marqueur de contrôle résistant, ce qui n'induit pas d'erreur sur la lecture du signal émis par le marqueur de détection du ligand de détection de l'analyte.
C) Importance de redéfinir la grille de lecture à l'issue du procédé d'analyse : comparaison des régions d'intérêt obtenues en chimiluminescence en définissant la grille de lecture par rapport à des positions théoriques versus en définissant la grille de lecture à partir du signal émis en fluorescence par le marqueur de contrôle résistant.
La localisation des spots réels (traits en pointillés) a été obtenue sur la base des signaux produits par le marqueur de contrôle résistant et appliquée sur l'image d'acquisition des signaux du marqueur de détection d'un ligand de l'analyte en chimiluminescence ici présentée à la figure 4. Le positionnement théorique des spots est indiqué en traits continus et montre clairement un décalage par rapport au positionnelement réel.
La comparaison entre les valeurs médiane en chimiluminescence des pixels situés sous les positions théoriques attendues (traits continus) et les positions réelles détectées (traits en pointillés) en fluorescence est montrée dans le tableau 7.

**Tableau 7 : Signal mesuré à partir de l'analyse de la région de présence théorique d'un spot versus signal mesuré à partir de l'analyse de la région de position réelle de ce même spot**

| | Spot 1 (haut à gauche) | Spot 3 (haut à droite) | Spot 5 (au milieu) | spot 9 (en bas à droite) |
|---|---|---|---|---|
| Position théorique | 319 | 301 | 58 | 3387 |
| Position réelle | 689 | 693 | 138 | 5233 |

L'analyse de la région contenant un spot produisant du signal fournit des résultats significativement plus élevés que la région de présence théorique de ce même spot. La quantification du signal et la justesse des résultats obtenus sont donc améliorées en se basant sur la grille de positionnement réel détecté grâce au marqueur de contrôle résistant.
D) Exemple de spot dégradé mesuré après un procédé d'analyse et qui aurait pu donner un faux résultat en l'absence de vérification de l'intégrité des spots.

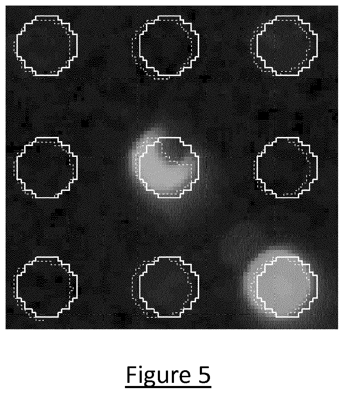

La localisation des spots réels (traits en pointillés) a été obtenue sur la base des signaux produits par le marqueur de contrôle résistant et appliquée sur l'image d'acquisition des signaux du marqueur de détection d'un ligand de l'analyte en chimiluminescence ici présentée (*cf.* *figure 5*). Le positionnement théorique des spots est indiqué en traits continus

Sur l'image (*cf.* *figure 5*), le spot situé au centre du puits est déformé. Le trait blanc discontinu qui l'entoure montre que le logiciel a détecté la forme réelle du spot, ce qui permet d'analyser son intégrité avant de valider le rendu du résultat. Dans ce cas, le paramètre de circularité de la forme réelle du spot permet d'éliminer ce spot et de ne pas rendre de valeur d'analyse fausse.

## Revendications

1. Procédé de détection d'au moins un analyte dans au moins un échantillon comprenant les étapes suivantes :
a) mettre un échantillon à analyser en présence du ou des spots d'un compartiment d'un support solide, ledit spot ou au moins un desdits spots comprenant au moins un marqueur de contrôle résistant et au moins un ligand de capture d'un analyte,
b) mettre en présence du ou des spots dudit compartiment au moins un ligand de détection d'un analyte, ledit ligand de détection d'un analyte étant couplé à un marqueur de détection direct ou indirect,
c) lorsqu'au moins un ligand de détection d'un analyte est couplé à un marqueur de détection indirect, mettre en présence du ou des spots dudit compartiment un rapporteur dudit marqueur de détection indirect,
d) lorsque le rapporteur utilisé à l'étape c) est couplé à un marqueur de détection indirect, mettre en présence du ou des spots dudit compartiment un rapporteur du marqueur de détection indirect couplé audit rapporteur utilisé à l'étape c),
e) détecter au moins un signal produit par au moins un marqueur de contrôle résistant dans ledit compartiment,
f) définir au moins une grille de lecture à partir de la localisation d'au moins un signal détecté à l'étape e),
g) détecter un signal produit par au moins un marqueur de détection d'un ligand de détection d'un analyte, et
h) lire le signal détecté à l'étape g) sur la grille de lecture définie à l'étape f) les étapes a) à d) étant réalisées avant les étapes e) à h),
ledit marqueur de contrôle résistant étant un marqueur dont on détecte un signal à la fin d'un procédé d'analyse,
ledit marqueur de contrôle résistant étant un fluorophore sélectionné dans le groupe consistant en une coumarine, une rhodamine, une carbopyronine, une oxazine, du benzopyrylium, une phycoérythrine et leurs dérivés.

2. Procédé selon la revendication 1, **caractérisé en ce que** le signal produit par au moins un marqueur de détection d'un ligand de détection d'un analyte est un signal luminescent.

3. Procédé selon la revendication 1 ou 2, **caractérisé en ce que** ledit, lesdits ou un desdits marqueurs de contrôle résistants est un fluorophore dont le spectre d'excitation ne recouvre pas le spectre d'émission du signal émis par ou correspondant au marqueur de détection dudit ou desdits ligands de détection d'un analyte et dont le spectre d'émission ne recouvre pas ou recouvre partiellement le spectre d'émission du ou correspondant au marqueur de détection dudit ou desdits ligands de détection d'un analyte.

4. Procédé selon l'une des revendications 1 à 3, **caractérisé en ce que** :
- le signal produit par au moins un marqueur de détection d'un ligand de détection d'un analyte est la lumière émise par un composé chimiluminescent obtenu par réaction d'une peroxydase avec le luminol, un analogue du luminol et/ou un dérivé du luminol ou d'un analogue du luminol, et
- le signal produit par le ou les marqueurs de contrôle résistant est une lumière émise en dehors des longueurs d'ondes allant de 375 nm à 550 nm.

5. Procédé de sélection d'un marqueur de contrôle résistant,
ledit marqueur de contrôle résistant étant un marqueur de contrôle dont on détecte un signal à la fin d'un procédé d'analyse,
ledit marqueur de contrôle résistant étant un fluorophore,
le dit procédé comprenant les étapes suivantes :
a) déposer un marqueur à tester à la surface d'un support solide, pour former au moins un spot,
b) saturer la surface du support solide,
c) sécher la surface du support solide,
d) réaliser au moins les étapes suivantes :
(i) réaliser une ou plusieurs étapes de lavage,
(ii) mettre le ou les spots en présence d'au moins un ligand de détection,
(iii) mettre le ou les spots en présence d'au moins un rapporteur,
(iv) mettre le ou les spots en présence d'au moins un substrat choisi parmi le luminol, un analogue du luminol, et/ou un dérivé du luminol,
e) sélectionner un marqueur qui produit un signal à l'issue de l'étape d).

6. Procédé de préparation d'un support solide pour la détection d'au moins un analyte dans un échantillon comprenant les étapes suivantes :
a) déposer, à la surface d'au moins un compartiment d'un support solide, un mélange comprenant au moins un marqueur de contrôle résistant et au moins un ligand de capture, pour obtenir un spot, ledit ou lesdits marqueurs de contrôle résistants étant des marqueurs qui restent au moins partiellement fixés au niveau dudit spot à la surface du support solide au cours de la mise en œuvre d'un procédé de détection d'au moins un analyte, de sorte à produire un signal détectable à l'issue dudit procédé de détection,
b) répéter l'étape a) n-1 fois, n étant un nombre entier supérieur ou égal à 1, pour obtenir n spots destinés à la détection d'un analyte à la surface dudit ou desdits compartiments,
ledit marqueur de contrôle résistant étant un marqueur dont on détecte un signal à la fin d'un procédé d'analyse
ledit marqueur de contrôle résistant étant un fluorophore sélectionné dans le groupe consistant en une coumarine, une rhodamine, une carbopyronine, une oxazine, du benzopyrylium, une phycoérythrine et leurs dérivés.

7. Procédé selon la revendication 6, comprenant en outre les étapes suivantes :
c) saturer la surface dudit ou desdits compartiments, et/ou
d) sécher la surface dudit ou desdits compartiments.

8. Procédé selon la revendication 6 ou 7, **caractérisé en ce que** ledit ou lesdits ligands de capture sont sélectionnés dans le groupe consistant en un anticorps, un antigène, un acide nucléique et leurs combinaisons.

9. Utilisation d'au moins un marqueur de contrôle résistant dans au moins un spot destiné à détecter un analyte pour sécuriser un procédé de détection d'au moins un analyte dans un échantillon, **caractérisée en ce qu'**elle comprend la lecture du signal produit par au moins un marqueur de détection d'un ligand de détection d'un analyte à détecter sur une grille de lecture définie à partir de la localisation du signal produit par ledit ou lesdits marqueurs de contrôle résistants et détecté à la fin du procédé d'analyse,
ledit ou lesdits marqueurs de contrôle résistant étant un marqueur dont on détecte un signal à la fin d'un procédé d'analyse,
ledit ou lesdits marqueurs de contrôle résistants étant un fluorophore sélectionné dans le groupe consistant en une coumarine, une rhodamine, une carbopyronine, une oxazine, du benzopyrylium, une phycoérythrine et leurs dérivés.

10. Procédé selon les revendications 1 à 8 ou utilisation selon la revendication 9, caractérisé(e) en ce que le fluorophore est couplé à une molécule porteuse.

## Patentansprüche

1. Verfahren zum Detektieren mindestens eines Analyten in mindestens einer Probe, aufweisend die folgenden Schritte:
a) Anordnen einer zu analysierenden Probe in Gegenwart des oder der Spots einer Kammer eines festen Trägers, wobei der Spot oder mindestens einer der Spots mindestens einen resistenten Kontrollmarker und mindestens einen Einfangliganden für einen Analyten aufweist,
b) Anordnen des oder der Spots der Kammer in Gegenwart mindestens eines Detektionsliganden eines Analyten, wobei der Detektionsligand eines Analyten an einen direkten oder indirekten Detektionsmarker gekoppelt ist,
c) wenn mindestens ein Detektionsligand eines Analyten an einen indirekten Detektionsmarker gekoppelt ist, Anordnen des oder der Spots der Kammer in Gegenwart eines Reporters des indirekten Detektionsmarkers,
d) wenn der im Schritt c) verwendete Reporter an einen indirekten Detektionsmarker gekoppelt ist, Anordnen des oder der Spots der Kammer in Gegenwart eines Reporters des indirekten Detektionsmarkers, der an den in Schritt c) verwendeten Reporter gekoppelt ist,
e) Detektieren mindestens eines Signals, das von mindestens einem resistenten Kontrollmarker in der Kammer erzeugt wird,
f) Definieren mindestens eines Leserasters ab der Lokalisierung mindestens eines in Schritt e) detektierten Signals,
g) Detektieren eines Signals, das von mindestens einem Detektionsmarker eines Detektionsliganden eines Analyten erzeugt wird, und
h) Lesen des in Schritt g) detektierten Signals auf dem in Schritt f) definierten Leseraster, wobei die Schritte a) bis d) vor den Schritten e) bis h) durchgeführt werden,
wobei der resistente Kontrollmarker ein Marker ist, von dem am Ende eines Analyseverfahrens ein Signal detektiert wird,
wobei der resistente Kontrollmarker ein Fluorophor ist, ausgewählt aus der Gruppe bestehend aus Cumarin, Rhodamin, Carbopyronin, Oxazin, Benzopyrylium, Phycoerythrin und Derivaten davon.

2. Verfahren gemäß Anspruch 1, **dadurch gekennzeichnet, dass** das von mindestens einem Detektionsmarker eines Detektionsliganden eines Analyten erzeugte Signal ein lumineszierendes Signal ist.

3. Verfahren gemäß Anspruch 1 oder 2, **dadurch gekennzeichnet, dass** der, die oder einer der resistenten Kontrollmarker ein Fluorophor ist, dessen Anregungsspektrum das Emissionsspektrum des Signals nicht abdeckt, das von dem Detektionsmarker des oder der Detektionsliganden eines Analyten emittiert wird oder diesem entspricht, und dessen Emissionsspektrum das Emissionsspektrum des Detektionsmarkers des oder der Detektionsliganden eines Analyten oder das Emissionsspektrum, das dem Detektionsmarker des oder der Detektionsliganden eines Analyten entspricht, nicht abdeckt oder teilweise abdeckt.

4. Verfahren gemäß einem der Ansprüche 1 bis 3, **dadurch gekennzeichnet, dass**:
- das von mindestens einem Detektionsmarker eines Detektionsliganden eines Analyten erzeugte Signal Licht ist, das von einer chemilumineszierenden Verbindung emittiert wird, die durch Reaktion einer Peroxidase mit Luminol, einem Luminol-Analogon und/oder einem Derivat von Luminol oder einem Luminol-Analogon erhalten wird, und
- das von dem oder den resistenten Kontrollmarkern erzeugte Signal Licht ist, das außerhalb des Wellenlängenbereichs von 375 nm bis 550 nm emittiert wird.

5. Verfahren zum Auswählen eines resistenten Kontrollmarkers,
wobei der resistente Kontrollmarker ein Kontrollmarker ist, von dem am Ende eines Analyseverfahrens ein Signal detektiert wird,
wobei der resistente Kontrollmarker ein Fluorophor ist,
wobei das Verfahren die folgenden Schritte aufweist:
a) Aufbringen eines zu testenden Markers auf die Oberfläche eines festen Trägers, um mindestens einen Spot auszubilden,
b) Sättigen der Oberfläche des festen Trägers,
c) Trocknen der Oberfläche des festen Trägers,
d) Durchführen mindestens der folgenden Schritte:
(i) Durchführen von einem oder mehreren Waschschritten,
(ii) Anordnen des oder der Spots in Gegenwart von mindestens einem Detektionsliganden,
(iii) Anordnen des oder der Spots in Gegenwart von mindestens einem Reporter,
(iv) Anordnen des oder der Spots in Gegenwart von mindestens einem Substrat, das aus Luminol, einem Luminol-Analogon und/oder einem Luminol-Derivat ausgewählt wird,
(e) Auswählen eines Markers, der am Ende des Schrittes (d) ein Signal erzeugt.

6. Verfahren zur Herstellung eines festen Trägers für das Detektieren mindestens eines Analyten in einer Probe, aufweisend die folgenden Schritte:
a) Aufbringen, auf die Oberfläche mindestens einer Kammer eines festen Trägers, eines Gemischs, das mindestens einen resistenten Kontrollmarker und mindestens einen Einfangliganden enthält, um einen Spot zu erhalten, wobei der oder die resistenten Kontrollmarker Marker sind, die während des Durchführens eines Verfahrens zum Detektieren mindestens eines Analyten zumindest teilweise an dem Spot auf der Oberfläche des festen Trägers fixiert bleiben, so dass am Ende des Detektionsverfahrens ein detektierbares Signal erzeugt wird,
b) n-1-maliges Wiederholen des Schrittes a), wobei n eine ganze Zahl größer oder gleich 1 ist, um n Spots, die zum Detektieren eines Analyten auf der Oberfläche der Kammer oder der Kammern vorgesehen sind, zu erhalten,
wobei der resistente Kontrollmarker ein Marker ist, von dem am Ende eines Analyseverfahrens ein Signal detektiert wird,
wobei der resistente Kontrollmarker ein Fluorophor ist, der aus der Gruppe bestehend aus Cumarin, Rhodamin, Carbopyronin, Oxazin, Benzopyrylium, Phycoerythrin und Derivaten davon ausgewählt wird.

7. Verfahren gemäß Anspruch 6, ferner die folgenden Schritte aufweisend:
c) Sättigen der Oberfläche der Kammer oder der Kammern, und/oder
d) Trocknen der Oberfläche der Kammer oder der Kammern.

8. Verfahren gemäß Anspruch 6 oder 7, **dadurch gekennzeichnet, dass** der oder die Einfangliganden aus der Gruppe bestehend aus einem Antikörper, einem Antigen, einer Nukleinsäure und deren Kombinationen ausgewählt werden.

9. Verwendung mindestens eines resistenten Kontrollmarkers in mindestens einem Spot, der vorgesehen ist, um einen Analyten zu detektieren, um ein Verfahren zum Detektieren mindestens eines Analyten in einer Probe zu sichern, **dadurch gekennzeichnet, dass** sie aufweist: das Lesen des Signals, das von mindestens einem Detektionsmarker eines Detektionsliganden eines zu detektierenden Analyten erzeugt wird, auf einem Leseraster, das ab der Lokalisierung des Signals definiert wird, das von dem oder den resistenten Kontrollmarkern erzeugt und am Ende des Analyseverfahrens detektiert wird,
wobei der oder die resistenten Kontrollmarker ein Marker sind, von dem am Ende eines Analyseverfahrens ein Signal detektiert wird,
wobei der oder die resistenten Kontrollmarker ein Fluorophor sind, ausgewählt aus der Gruppe bestehend aus Cumarin, Rhodamin, Carbopyronin, Oxazin, Benzopyrylium, Phycoerythrin und Derivaten davon.

10. Verfahren gemäß den Ansprüchen 1 bis 8 oder Verwendung gemäß Anspruch 9, **dadurch gekennzeichnet, dass** der Fluorophor an ein Trägermolekül gekoppelt ist/wird.

## Claims

1. Method for detecting at least one analyte in at least one sample comprising the following steps:
a) bringing together a sample to be analysed and the spot or spots of a compartment of a solid support, said spot or at least one of said spots comprising at least one resistant control marker and at least one analyte-capture ligand,
b) bringing together the spot or spots of said compartment and at least one analyte-detecting ligand, said analyte-detecting ligand being coupled with a direct or indirect detection marker,
c) when at least one analyte-detecting ligand is coupled with an indirect detection marker, bringing together the spot or spots of said compartment and a reporter of said indirect detection marker,
d) when the reporter used in step c) is coupled with an indirect detection marker, bringing together the spot or spots of said compartment and a reporter of the indirect detection marker coupled with said reporter used in step c),
e) detecting at least one signal produced by at least one resistant control marker in said compartment,
f) defining at least one reading grid from the location of at least one signal detected in step e),
g) detecting a signal produced by at least one detection marker of an analyte-detecting ligand, and
h) reading the signal detected in step g) on the reading grid defined in step f),
the steps a) to d) being implemented before steps e) to h),
said resistant control marker being a marker, from which a signal is detected at the end of an analysis process,
said resistant control marker being a fluorophore selected from the group consisting of a coumarin, a rhodamine, a carbopyronine, an oxazine, benzopyrylium, a phycoerythrin and their derivatives.

2. Method according to claim 1, **characterised in that** the signal produced by at least one detection marker of an analyte-detecting ligand is a luminescent signal.

3. Method according to claim 1 or 2, **characterised in that** said resistant control marker, said resistant control markers or one of said resistant control markers is a fluorophore, the excitation spectrum of which does not cover the emission spectrum of the signal emitted by or corresponding to the detection marker of said analyte-detecting ligand or of said analyte-detecting ligands and the emission spectrum of which does not cover or covers partially the emission spectrum of or corresponding to the detection marker of said analyte-detecting ligand or of said analyte-detecting ligands.

4. Method according to one of the claims 1 to 3, **characterised in that**:
- the signal produced by at least one detection marker of an analyte-detecting ligand is the light emitted by a chemiluminescent compound obtained by reaction of a peroxidase with luminol, an analogue of luminol and/or a derivative of luminol or an analogue of luminol, and
- the signal produced by the resistant control marker or resistant control markers is a light emitted outside the wavelengths going from 375 nm to 550 nm.

5. Method for selecting a resistant control marker,
said resistant control marker being a control marker, from which a signal is detected at the end of an analysis process,
said resistant control marker being a fluorophore,
said method comprising the following steps:
a) placing a marker to be tested on the surface of a solid support, in order to form at least one spot,
b) saturating the surface of the solid support,
c) drying the surface of the solid support,
d) implementing at least the following steps:
(i) implementing one or more washing steps,
(ii) bringing together the spot or spots and at least one detection ligand,
(iii) bringing together the spot or spots and at least one reporter,
(iv) bringing together the spot or spots and at least one substrate chosen amongst luminol, an analogue of luminol, and/or a derivative of luminol,
e) selecting a marker which produces a signal at the end of step d).

6. Method for preparing a solid support for detecting at least one analyte in a sample comprising the following steps:
a) placing, on the surface of at least one compartment of a solid support, a mixture comprising at least one resistant control marker and at least one capture ligand, in order to obtain a spot, said resistant control marker or said resistant control markers being markers which remain at least partially fixed at the level of said spot on the surface of the solid support in the course of implementing a method for detecting at least one analyte, so as to produce a signal detectable at the end of said detection process,
b) repeating step a) n-1 times, n being a whole number greater than or equal to 1, in order to obtain n spots intended for detecting an analyte on the surface of said compartment or said compartments,
said resistant control marker being a marker, from which a signal is detected at the end of an analysis process,
said resistant control marker being a fluorophore selected from the group consisting of a coumarin, a rhodamine, a carbopyronine, an oxazine, benzopyrylium, a phycoerythrin and their derivatives.

7. Method according to claim 6, comprising furthermore the following steps:
c) saturating the surface of said compartment or said compartments, and/or
d) drying the surface of said compartment or said compartments.

8. Method according to claim 6 or 7, **characterised in that** said capture ligand or said capture ligands are selected from the group consisting of an antibody, an antigen, a nucleic acid and their combinations.

9. Use of at least one resistant control marker in at least one spot intended to detect an analyte in order to safeguard a method for detecting at least one analyte in a sample, **characterised in that** it comprises the reading of the signal produced by at least one detection marker of an analyte-detecting ligand to be detected on a reading grid defined from the location of the signal produced by said resistant control marker or said resistant control markers and detected at the end of the analysis process,
said resistant control marker or said resistant control markers being a marker, from which a signal is detected at the end of an analysis process,
said resistant control marker or said resistant control markers being a fluorophore selected from the group consisting of a coumarin, a rhodamine, a carbopyronine, an oxazine, benzopyrylium, a phycoerythrin and their derivatives.

10. Method according to claims 1 to 8 or use according to claim 9, **characterised in that** the fluorophore is coupled with a carrier molecule.
